# EUROPEAN PATENT SPECIFICATION

(11) **EP 1 644 007 B1**
(45) Date of publication and mention of the grant of the patent: **10.10.2012**
(21) Application number: 04737755.1
(22) Date of filing: 31.05.2004
(51) Int. Cl.: A61K 31/575, A23L 1/30, A23L 1/0528

(54) **COMPOSITION COMPRISING ONE OR MORE PHYTOSTEROLS AND/OR PHYTOSTANOLS AND GLUCOMANNAN AND USES OF THE COMPOSITION IN TREATING LIPID DISORDERS IN INDIVIDUALS WITH AND WITHOUT TYPE II DIABETES**
ZUSAMMENSETZUNG MIT EIN ODER MEHR PHYTOSTEROLEN UND/ODER PHYTOSTANOLEN UND GLUCOMANNAN UND ANWENDUNGEN DER ZUSAMMENSETZUNG BEI DER BEHANDLUNG VON LIPIDSTÖRUNGEN BEI PERSONEN MIT UND OHNE TYP II DIABETES
COMPOSITION COMPRENANT UN OU PLUSIEURS PHYTOSTEROLS ET/OU PHYTOSTANOLS ET GLUCOMANNAN, ET UTILISATIONS DE CETTE COMPOSITION DANS LE TRAITEMENT DES TROUBLES LIPIDIQUES CHEZ DES INDIVIDUS SOUFFRANT OU NON DU DIABETE DU TYPE II

(30) Priority: 31.05.2003 US 449729
(43) Date of publication of application: 12.04.2006
(73) Proprietor: Pharmachem Laboratories, Inc., Kearny, New Jersey 07032 (US)
(72) Inventor: ZAWISTOWSKI, Jerzy, Port Moody, British Columbia V3H 4K6 (CA); JONES, Peter, J., Baie D'Urfe, Quebec H9X 2V4 (CA)
(74) Representative: Jones, Helen M.M.
(86) International application number: PCT/CA2004/000807
(87) International publication number: WO 2004/105770

(56) References cited:
- EP-A- 1 302 113
- WO-A-02/082929
- WO-A-03/024237
- US-B1- 6 365 176
- DATABASE BIOSIS [Online] BIOSCIENCES INFORMATION SERVICE, PHILADELPHIA, PA, US; 2004, YOSHIDA MAKIKO ET AL: "Glucomannan and plant sterols decrease plasma cholesterol levels in an additive manner in type 2 diabetic and non-diabetic subjects" XP002294797 Database accession no. PREV200400286623 & FASEB JOURNAL, vol. 18, no. 4-5, 2004, pages Abst. 600.4 URL-http://ww, FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638
- DATABASE WPI Section Ch, Week 200364 Derwent Publications Ltd., London, GB; Class B04, AN 2003-674119 XP002294798 & JP 2003 113394 A (UNITIKA LTD) 18 April 2003 (2003-04-18)

## Description

### FIELD OF THE INVENTION

This present invention relates to compositions useful in the field of lipid disorders.

### BACKGROUND OF THE INVENTION

While recent advances in science and technology are helping to improve quality and add years to human life, the prevention of atherosclerosis, the underlying cause of cardiovascular disease ("CVD") has not been sufficiently addressed. In fact, cardiovascular diseases account for more deaths annually than any other disease, including all forms of cancer combined¹. In the USA alone, more than one million heart attacks occur each year and more than half a million people die as a result. This enormous toll has necessitated continued research to determine the causes of CVD and means by which it can be prevented and treated.

The primary cause of CVD is atherosclerosis, a disease characterized by the deposition of lipids, including cholesterol, in the arterial vessel wall resulting in a narrowing of the vessel passages and ultimately a hardening of the vascular system. Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree ^{1,2}. A total cholesterol in excess of 225-250 mg/dl is associated with significantly elevated risk of CVD, including vascular disease. Overviews have indicated that a 1% reduction in a person's total serum cholesterol yields a 2% reduction in risk of a coronary artery event⁴. Statistically, a 10% decrease in average serum cholesterol (e.g. from 6.0 mmol/L to 5.3 mmol/L) may result in the prevention of 100,000 deaths in the United States annually⁵.

Cholesteryl esters are a major component of atherosclerotic lesions and the major storage form of cholesterol in arterial wall cells. Formation of cholesteryl esters is also a step in the intestinal absorption of dietary cholesterol through homeostatic control mechanisms. These control mechanisms involve the inter-related regulation of dietary cholesterol, cholesterol biosynthesis and catabolism of cholesterol-containing plasma lipoproteins. Cholesterol biosynthesis and catabolism occur primarily in the liver and hence, it is a prime determinant of plasma cholesterol levels.

Lipoproteins are complexes of lipids and proteins held together by non-covalent bonds. Each type of lipoprotein class has a characteristic mass, chemical composition, density and physiological role. Irrespective of density or particle size, circulating lipids consist of a core of cholesteryl esters and triglycerides, and an envelope of phospholipids, free cholesterol and apolipoproteins. The apolipoproteins are involved in the assembly and secretion of the lipoprotein, provide structural integrity, activate lipoprotein-modifying enzymes, and are the ligand for a large assortment of receptors and membrane proteins. Lipoprotein classes found in plasma include HDL, LDL, intermediate density lipoproteins (IDL) and very low density lipoproteins (VLDL).

Each type of lipoprotein has a characteristic apolipoprotein composition or ratio. The most prominent apolipoprotein in HDL is apolipoprotein-Al (apo-Al), which accounts for approximately 70% of the protein mass, with apo-AII accounting for another 20%. The ratio of acoA-I to acoA-II may determine HDL functional and anti-atherogenic properties. Circulating HDL particles consist of a heterogeneous mixture of discoidal and spherical particles with a mass of 200 to 400 kilo-daltons and a diameter of 7 to 10 nm.

HDL is one of the major classes of lipoproteins that function in the transport of lipids in plasma, and has multiple functions within the body, including reverse cholesterol transport, providing the cholesterol molecule substrate for bile acid synthesis, transport of clusterin, transport of paraoxanase, prevention of lipoprotein oxidation and selective uptake of cholesterol by adrenal cells. The major lipids associated with HDL include cholesterol, cholesteryl ester, triglycerides, phospholipids and fatty acids. HDL is anti-atherogenic.

The atherosclerotic process begins when LDL becomes trapped within the vascular wall.

Oxidation of this LDL results in the binding of monocytes to the endothelial cells lining the vessel wall. These monocytes are activated and migrate into the endothelial space where they are transformed into macrophages, leading to further oxidation of the LDL. The oxidized LDL is taken up through the scavenger receptor on the macrophage, leading to the formation of foam cells. A fibrous cap is generated through the proliferation and migration of arterial smooth muscle cells, thus creating an atherosclerotic plaque.

HDL is essential for the transport of cholesterol from extra-hepatic tissues to the liver, where it is excreted into bile as free cholesterol or as bile acids that are formed from cholesterol. The process requires several steps. The first is the formation of nascent or pre-beta HDL particles in the liver and intestine. Excess cholesterol moves across cell membranes into the nascent HDL through the action of the ABCA transporter. Lecithin cholesterol acyl transferase (LCAT) converts the cholesterol to cholesteryl ester and the subsequent conversion of nascent HDL to mature HDL. Esterified cholesterol is then transferred by cholesteryl ester transfer protein (CETP) from HDL to apolipoprotein-B containing lipoproteins, which are taken up by numerous receptors in the liver. Nascent HDL is regenerated via hepatic triglyceride lipase and phospholipid transfer protein and the cycle continues. In addition to the cholesterol removed from peripheral cells, HDL accepts cholesterol from LDL and erythrocyte membranes. Another mechanism of reverse cholesterol transport may involve passive diffusion of cholesterol between cholesterol-poor membranes and HDL or other acceptor molecules.

HDL protects against the development of atherosclerosis both through its role in reverse cholesterol transport and possibly by impeding LDL oxidation. Several HDL-associated enzymes are involved in the process. Paroxonase (PON1), LCAT, and platelet activating factor acetylhydrolase (PAFAH) all participate by hydrolyzing phospholipid hydroperoxides generated during LDL oxidation and act in tandem to prevent the accumulation of oxidized lipid in LDL. These enzymes are responsible for the anti-oxidative and anti-inflammatory properties of HDL. Studies have shown that a low plasma concentration of HDL cholesterol is a significant risk factor for the development of atherosclerosis and that high levels are protective.

The liver is the major organ responsible for synthesis and secretion of VLDLs, which, as noted above, are metabolized to LDL in circulation. LDLs are the predominant cholesterol carrying lipoproteins in plasma and hence an increase in their concentration is directly correlated with atherosclerosis. Simply put, when intestinal cholesterol absorption is reduced, by any means, less cholesterol is delivered to the liver. As a result, VLDL production is reduced and there is a concomitant increase in hepatic clearance of plasma cholesterol, mostly in the form of LDL.

Accordingly, cholesterol acts on three different levels to regulate its own synthesis. Firstly, it suppresses endogenous cholesterol synthesis by inhibiting the enzyme HMG CoA reductase. Secondly, it activates LCAT. Thirdly, it regulates the synthesis of the LDL-receptor ensuring that a cell already having a sufficient amount of cholesterol will not take up additional cholesterol.

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional western-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, Including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

Over forty years ago, Eli Lilly marketed a sterol preparation from tall oil and later from soybean oil called Cytellin™ which was found to lower serum cholesterol by about 9% according to one report⁶. Various subsequent researchers have explored the effects of sitosterol preparations on plasma lipid and lipoprotein concentrations⁷ and the effects of sitosterol and campesterol from soybean and tall oil sources on serum cholesterols⁸. A composition of phytosterols which has been found to be highly effective in lowering serum cholesterol is disclosed in US Patent Serial No. 5,770,749 to Kutney et al. and comprises no more than 70% by weight beta-sitosterol, at least 10% by weight campesterol and stigmastanol (beta-sitostanol). It is noted in this patent that there is some form of synergy between the constituent phytosterols, affording even better cholesterol-lowering results than had been previously achieved.

Many other phytosterol-based compounds and compositions have been developed over the last decade, with a view either to lowering serum LDL cholesterol, increasing serum HDL cholesterol and preventing other significant risk factors for CVD.

Diabetes mellitus is a heterogeneous primary disorder of carbohydrate metabolism with multiple etiologic factors that generally involve Insulin deficiency or Insulin resistance or both. Type I, or juvenile onset, or insulin-dependent diabetes mellitus, is present in patients with little or no endogenous insulin secretory capacity. These patients develop extreme hyperglycemia (glucose accumulation in the bloodstream) and are entirely dependent on exogenous insulin therapy for immediate survival. Type 2 also called adult onset, or non-insulin-dependent diabetes mellitus ("NIDDM"), occurs in patients who retain some endogenous insulin secretory capacity, however the great majority of them are both insulin deficient and insulin resistant. Insulin resistance can be due to insufficient Insulin receptor expression, reduced insulin-binding affinity, or any abnormality at any step along the insulin signaling pathway.

Diabetes mellitus is one of the major causes of mortality and morbidity in the World. In the US alone, the disease affects close to 5% of the US population and about 10% of those over 60. It has been estimated that there are about 12 million diabetics in the US both diagnosed and undiagnosed. This number grows annually, and only in past decade, the US has seen a dramatic 33% rise in diabetes coupled with increasing obesity and inappropriate lifestyle. It is worth noting that by far the largest percentage (90%) of diabetics has Type 2 variety.

Diabetes mellitus also affects over 30 million people in China, and the number of diabetics is increasing by 5% per year. Only about 5% are Type I diabetics, the remainder having the Type 2 variety.

Carbohydrates from food are broken down in the intestine to glucose and other simple sugars.

These sugars are absorbed into the bloodstream and carried (along with other nutrients) to all the cells of the body. But in order to take up glucose from the blood, the cells need insulin -- a hormone made in the pancreas. The pancreas, a large gland located behind the stomach, has multiple functions. It produces digestive enzymes - proteins that help break down food In the intestine. It also contains specialized groups of cells called "islets of Langerhans". These islet cells are of several types, each producing a different hormone. There are two main hormones that regulate blood glucose levels - insulin and glucagon. Both are produced in the islets of Langerhans - glucagon by alpha cells, and insulin by beta cells.

As glucose pours into the bloodstream after a meal, the rising blood glucose level signals the beta cells to secrete insulin. Insulin (along with the glucose) is carried by the bloodstream to cells throughout the body. Various body tissues -- especially muscle, liver, and fat -- have specialized molecules called insulin receptors on their cell surfaces. Insulin binds to these receptors, like a key in a lock - opening channels that allow glucose to enter the cells.

Once glucose is inside the cells, it can be used for energy and growth. Excess glucose is stored in the liver in the form of a complex carbohydrate called glycogen. Meanwhile, as blood glucose levels fall, insulin secretion slows down. When the blood glucose level starts to get low, it signals the alpha cells to secrete glucagons, a peptide hormone. Glucagon, in turn, signals the liver to convert glycogen back into glucose - a process called glycogenolysis. This prevents the blood glucose level from dropping too low to ensure that the body's cells have a steady supply of glucose between meals. Glucagon also stimulates the liver to make new glucose out of other nutrients, such as amino acids (protein building blocks) by a process called gluconeogenesis thereby ensuring a backup source of glucose until the next meal.

The interaction of glucose, insulin, and glucagon normally ensures that blood glucose levels stay within certain limits. With diabetic individuals, however, this delicate balance is upset.

To date, insulin is the primary mode of therapy in all patients with Type I and in many with Type 2 diabetes.

Type 2 diabetes usually begins in adulthood (typically after age 40), but also can occur in younger people. Generally, in people having this condition, the pancreas produces insulin but the body's cells are unable to respond to it effectively. This is called insulin resistance. A person can have insulin resistance without diabetes, as long as there is enough insulin to overcome the resistance. But if insulin resistance continues to increase and/or insulin production falls below the amount needed to compensate, diabetes will develop.

Most type 2 diabetes can be treated with oral medications that boost pancreatic insulin secretion and/or lower insulin resistance. But over time, the pancreatic beta cells may become less and less responsive thereby secreting less insulin when blood glucose levels are high. Eventually, the person may need insulin injections to help control blood glucose levels.

Abnormal glucose tolerance and insulin resistance are related to multiple cardiovascular risk factors, especially reduced HDL, elevated plasma triglycerides and hypertension⁹. When clustered, these abnormalities increase the risk of CVD morbidity and mortality, an effect which is independent of other risk factors¹⁰. Hyperglycemia and diabetes are both significant independent risk factors for CVD mortality¹¹. Since people with diabetes have almost twice the risk of dying from CVD as those in the general US population¹², the control of high glucose levels and the significant CVD risk factors represents the most effective approach to prevention¹³.

Accordingly, controlling CVD risk factors, including serum cholesterol, is critical to both diabetic and non-diabetic individuals alike.

US6365176 discloses nutritional supplements for patients with type 2 diabetes mellitus for lipodystrophy comprising 0.5-1 g of konjac mannan and 0.5-4 g of plant sterol.

WO03/024237 discloses an oil composition that comprises 0-5% of konjac and 0-10% phytosterol.

EP1302113 discloses physiologically functional foods or cosmetics containing sphingoglycolipids and processes for their production.

WO02/082929 discloses compositions comprising β-glucans and plant sterols and/or stanols.

JP2003113394 discloses the use of an extract of the tuberous root of devil's tongue comprising konjac and a vegetable sterol derived therefrom.

### SUMMARY OF THE INVENTION

The present invention provides a composition for incorporation into foods, beverages, nutritional or vitamin supplements, and nutraceuticals, which comprises one or more phytosterols and/or phytostanols and additionally, glucomannan, wherein the phytosterol and/or phytosterol in the composition comprises 67.3% sitosterol, 10.8% sitostanol, 8.2% campesterol, 1.6% campestanol and 12.1% of others.

The present invention further provides a use as defined in claim 4

It has been found that the compositions of the present invention exhibit superior activity in both for treating or preventing CVD and its underlying conditions, particularly in one or more of the following: lowering serum LDL cholesterol, increasing serum HDL cholesterol and increasing serum triglycerides. There may be at least an additive prophylactic and/or treatment effect, in all of these respects. Equally importantly, it is believed that when the components of this composition are combined, a lower dosage each selected component may be required to achieve these desired effects. This is important due to the documented side-effects of bulk glucomannan administration. These effects and other significant advantages will become apparent herein below.

### BRIEF DESCRIPTION OF THE DRAWINGS

Figure 1 is a graph showing the weekly changes in total cholesterol levels;
Figure 2 is a graph showing the weekly changes in of low-density lipoprotein cholesterol levels; and
Figure 3 is a graph showing the blood glucose changes during 2 hour 50mg glucose tolerance test.

### PREFERRED EMBODIMENTS OF THE INVENTION

The following detailed description is provided to aid those skilled in the art in practising the present invention. However, this detailed description should not be construed so as to unduly limit the scope of the present invention. Modifications and variations to the embodiments discussed herein may be made by those with ordinary skill in the art.

As used herein, "animal" means any member of the animal kingdom, including preferably humans.

As used herein, "food" means any safe, ingestible product for animal use, including human use, and includes "functional foods", "designer foods" and "pharmafoods".

As used herein, "functional food" means a product that is similar in appearance to conventional foods that is consumed as part of a usual diet, but which has demonstrated physiological benefits and/or reduces the risk of disease beyond basic nutritional functions.

As used herein, "nutraceutical" means a non-pharmaceutical product prepared in the form of pills, powders, potions and in other medicinal forms not generally associated with food but which has a physiological benefit or provides protection against disease.

Anywhere in the world, nutraceuticals, functional foods, designer foods, and pharmafoods are synonyms for food or food ingredients considered to provide medical or health benefits, including the prevention and treatment of disease.

According to the present invention, there is provided a composition for incorporation into foods, beverages, nutritional or vitamin supplements, and nutraceuticals, which comprises one or more phytosterols and/or phytostanols and glucomannan.

The elements of the composition will be described in more detail below.

### Phytosterols/Phytostanols

As used herein, the term "phytosterol" includes all sterols without limitation, for example: sitosterol, campesterol, stigmasterol, brassicasterol (including dihydrobrassicasterol), desmosterol, chalinosterol, poriferasterol, clionasterol, ergosterol, coprosterol, codisterol, isofucosterol, fucosterol, clerosterol, nervisterol, lathosterol, stellasterol, spinasterol, chondrillasterol, peposterol, avenasterol, isoavenasterol, fecosterol, pollinastasterol, and all natural or synthesized forms and derivatives thereof, including isomers. The term "phytostanol" refers to saturated or hydrogenated sterols including all natural or synthesized forms and derivatives thereof, and isomers. It is to be understood that modifications to the phytosterols and phtostanols i.e. to include side chains also falls within the purview of this invention. For example, the purview of this invention clearly includes 24 beta-ethylsitostanol, 24-alpha-ethyl-22-dehydrositostanol. It is also to be understood that, when in doubt throughout the specification, and unless otherwise specified, the term "phytosterol" encompasses both sterol and stanol. In a most preferred form, the sterol is in its saturated form and is a sitostanol, preferably beta-sitostanol.

These sterols and stanols for use in accordance with this invention may be procured from a variety of natural sources. For example, they may be obtained from the processing of plant oils (including aquatic plants) such as corn oil and other vegetable oils, wheat germ oil, soy extract, rice extract, rice bran, rapeseed oil, sunflower oil, sesame oil and fish (and other marine-source) oils. They may also be derived from fungi, for example ergosterol. Accordingly, the present invention is not to be limited to any one source of sterols. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749, incorporated herein by reference.

Phytosterols and phytostanols, as used herein, may be in their free or esterified forms i.e. optionally, the phytosterol or phytostanol may be esterified prior to formation of the composition described herein. This esterification step renders the phytosterols and/or phytostanols more soluble in fats and oils which may, in some instances, facilitate the incorporation of the composition into various delivery systems.

To form phytosterol and/or phytostanol esters, many methods are known in the art. For example, one or more suitable aliphatic acids or their esters with low boiling alcohols may be condensed with the selected phytosterol and/or phytostanol. A wide variety of aliphatic acids or their esters may be used successfully and include all aliphatic acids consisting of one or more alkyl chains with one or more terminal carboxyl groups. These aliphatic acids may be natural of synthetic and are represented by the following chemical formulae:
a) R1-COOH (monocarboxylic acid) wherein:
   R1 is an unbranched saturated alky group, represented by CH3-, CH3CH2- or CH3(CH2)nCH2- WHERE n=3-25; or
   R1 Is a branched saturated alkyl group represented by CnH2n+1-where n=1-25 is the number of carbon atoms contained in the group R1; the branching typically refers, but is not limited to one or more methyl group side chains (branches); or
   R1 is an unbranched or branched unsaturated alkyl group, represented by the formula CnH2n-2m+1, where n=1-25 is the number of carbon atoms in R1 and m=degree of unsaturation; or
b) HOOC-R2-COOH is a dicarboxylic acid wherein:
   R2 is an unbranched saturated alkly group, represented by - CH2-, or -CH2CH2-, or - CH2(CH2)nCH2 where n=3-25; or
   R2 is a branched saturated alkyl group represented by -CnH2n- where n=1-25 is the number of carbon atoms contained in the group R2; the branching typically refers, but is not limited to, one or more methyl group side chains (branches); or
   R2 is an unbranched or branched unsaturated alkyl group, represented by the formula CnH2n-2m, where n=1-25 is the number of carbon atoms in R2 and m=degree of unsaturation; or
c) a tricarboxylic acid represented by the formula:

   HOOC-R3-COOH COOH

   wherein, in this formula:
   R3 is a branched saturated alkyl group represented by -CnH2n-1- where n=1-25 is the number of carbon atoms contained in the group R3; the branching typically refers, but is not limited to, one or more methyl group side chains (branches); or
   R3 is a branched unsaturated alkyl group, represented by CnH2n-2m-1- wherein n=1-25 is the number of carbon atoms in R3 and m= the degree of unsaturation; or
d) a mono-. di-, or tricarboxylic acid as defined above, which may contain one, two or three hydroxyl groups in the molecule.

In a preferred form, the add is either a straight-chain or branched unsaturated or saturated, aliphatic or aromatic acid. More preferably, the acids are selected, inter alia, from the following list: valeric acid, isovaleric acid, sorbic acid, isocaproic acid, lauric acid, myrestic acid, palmitic acid, stearic acid, caproic acid, ascorbic acid, arachidic acid, behenic acid, hexacosanoic acid, octacosanoic acid, pentadecanoic acid, erucic acid, linoleic acid, linolenic acid, arachidonic acid, acetic acid, citric add, tartaric acid, palmitoleic acid and oleic acid. The most preferable fatty acids within the scope of the present invention are linoleic acid, linolenic acid and arachidonic acid which may be obtained from natural sources such as safflower oil, sunflower oil, olive oil and corn oil (linoleic acid), safflower oil, sunflower oil, olive oil and jojoba oil (linolenic acid and arachidonic acid) and rapeseed oil (erucic acid).

Other aromatic acids are clearly contemplated within the scope of the present invention.

A particular advantage in using fatty acids to form esterifed phytosterols or phytostanols i.e. saturated fats, in accordance with the present invention lies in the fact that saturated fats increase lipoprotein lipase activity. The activity of this latter enzyme reduces visceral fat formation.

To form a phytosterol ester in accordance with the present invention, the selected phytosterol and acid or its ester with volatile alcohol may be mixed together under reaction conditions to permit condensation of the phytosterol with the acid to produce an ester. A most preferred method of preparing these esters which is widely used in the edible fat and oil industry is described in US Patent Serial No. 5,502,045 (which is incorporated herein by reference). As no substances other than the free phytosterol, a fatty acid ester or mixture thereof and an interesterification catalyst like sodium ethylate are used, the technique is highly suitable for preparing products ultimately for human consumption. In overview, this preferred method, adapted for use within the present invention, comprises heating the phytosterol(s) with a vegetable oil fatty acid ester (preferably a methyl ester) at a temperature from 90-120°C and subsequently adding a suitable catalyst such as sodium ethylate. The catalyst is then removed/destroyed by any one of the techniques known in the art e.g. adding water and/or filtration/centrifugation.

Another method which may be used in accordance with the present invention is described in US Patent Serial No. 4,588,717, which is also incorporated herein by reference. A preferred method is to mix the phytosterol and the fatty acid together bringing the mixture to a temperature of from about 15°C to about 45°C at about atmospheric pressure for approximately one to three hours.

Accordingly, it is to be understood that the widest possible definition is to be accorded to the terms "phytosterol" and "phytostanol" as used herein, including, but not limited to: free phytosterols and phytostanols, esterified phytosterols and phytostanols with aliphatic or aromatic acids (thereby forming aliphatic or aromatic esters, respectively), phenolic acid esters, cinnamate esters, ferulate esters, phytosterol and phytostanol glycosides and acylated glycosides or acylglycosides.

In another preferred embodiment, the sterol and/or stanol is incorporated into a micelle prior to or after combining with glucomannan. This micelle can be produced using lecithin or any other suitable emulsifying agent and using techniques known and applied widely in the art.

### Glucomannan

Glucomannan (also referred to, interchangeably, as glucomanna, konjac, konjac fiber, konjac glucomanna, konjak fibre glucomannan, konjac glucomannan, and manna) is a water soluble dietary fibre derived primarily from the konjak root of the perennial Konjak plant (*Amorphophallus konjak C Koch*). Konjak root is a perennial plant unique to Asia, and is cultivated extensively in Japan. The fresh Konjac tuber contains an average of 13% dry matter of which approximately 70% is glucomannan (the remaining 30% being starch). There are other sources of glucomannan, such as, for example, the aloe plant, yeast and fenugreek, however, while these sources may be used, the preferred source is the Konjak tuber.

"Konnyaku", which is made from the tuber of Konjak, has been used in traditional Japanese medicine and foods for hundreds of years, the primary component of which is glucomannan. Edible konnyaku is made from konjak flour, which is derived from the tuber.

Glucomannan is a high molecular weight polysaccharide (200,000-2,000,000 Daltons) consisting of repeating units of beta-D glucose and beta-D mannose joined In 1,4 linkages

It has the highest molecular weight and one of the strongest viscosity of any other dietary fibre.¹⁴. Like other dietary fibres, glucomannan is considered a "bulk forming" laxative and has been used to treat constipation¹⁵. It has been shown to lower serum glucose levels in diabetic patients¹⁶ and to assist in pregnancy related diabetes¹⁷. Glucomannan, taken as a supplement, has been shown to have effects in lowering lipids and blood pressure¹⁸ and glycemia¹⁹.

The tubers of konjak have been traditionally pounded to yield a powder for making into noodles or gelatin for adding to stews, sauces and soups. Many, more contemporary techniques, are known in the art for separating glucomannan from konjak flour. In one, konjak flour is boiled in water, treated with Fehling's solution to convert the glucomannan to its copper complex, and the latter is decomposed again into the glucomannan after purification. This method is disclosed in more detail in J. Agri. Chem Soc. Japan,6,991-995 (1930). In another technique, konak flour is extracted with water, impurities are removed by precipitating with ethanol and redissolving the precipitate in water several times and drying the obtained precipitate to obtain pure glucomannan. This technique is described in Bull. Chem.Soc.Japan 49,298-322 (1927). There are varied and numerous other techniques known in the art and the preparation of the composition of the present invention is not limited to any one particular method of extracting and purifying glucomannan.

### Methods of Use

There is disclosed herein a method of treating or preventing cardiovascular disease and its underlying conditions including atherosclerosis, hyperlipidemic conditions, dyslipidemia, hypoalphalipoproteinemia, hypertension, hypercholesterolemia and visceral obesity in an animal which comprises administering a non-toxic and therapeutically effective amount of a composition comprising one or more phytosterols and/or phytostanols and glucomannan.

Further disclosed is a method of achieving at least one of the following prophylactic and treatment goals in an animal: lowering serum LDL cholesterol, increasing serum HDL cholesterol, decreasing serum triglycerides, decreasing weight gain and increasing weight loss and treating the complications and conditions associated with and which may be causal to Type 2 diabetes, which comprises administering to the animal a non-toxic and therapeutically effective amount of the novel composition.

This invention further comprises any of the claimed compositions for these indications, particularly, but not exclusively in the form of foods, beverages, nutraceuticals, nutritional supplements, and vitamin supplements.

In particular, the compounds of the present invention have been found to be especially useful in addressing at least two significant factors contributing to the multi-factorial presentation of cardiovascular disease: elevated LDL levels and elevated total cholesterol cholesterol levels.

The desired effects described herein may be achieved in a number of different ways. As noted above, the compositions of the present invention may be administered by any conventional means available for use in conjunction with nutraceuticals, foods including functional foods, beverages, and the like.

The term "therapeutically effective" is intended to qualify the amount of the composition taken by or administered to the animal, in particular the human, in order to achieve one or more of the following prophylactic and/or treatment goals:
a) lowering serum LDL cholesterol;
b) increasing serum HDL cholesterol;
c) decreasing serum triglycerides levels;
d) treating conditions associated with CVD generally;
e) treating atherosclerosis;
f) treating hypercholesterolemia;
g) treating a hyperlipidemic condition;
h) treating hypertension;
i) decreasing cholesterol synthesis;
j) decreasing weight gain;
k) increasing weight loss;
l) preventing, reducing, eliminating or ameliorating a dyslipidemic condition or disorder;
m) preventing, reducing, eliminating or ameliorating hypercholesterolemia, hypoalphalipoproteinemia,
n) preventing, reducing, eliminating or ameliorating the development of atherosclerotic lesions;
o) preventing, reducing, eliminating or ameliorating any condition, disease or disorder which has as its basis or which is exacerbated by a deficiency in plasma HDL, or excess of either LDL, VLDL, Lp(a), beta-VLDL, IDL or remnant lipoproteins;
p) treating diabetes mellitus and conditions associated with diabetes mellitus;
q) improving glucose tolerance;
r) regulating serum glucose levels; and
s) enhancing cellular insulin sensitivity.

It is preferred that at least 0.8 grams of phytosterol/phytostanol and up to 13 grams of glucomannan be provided in the composition, in one or more forms, for dally administration, based on a 70kg individual human. More preferably, between 1.0g and 3 grams of phytosterol/phytostanol and from approximately 2-10 grams of glucomannan can be provided in the composition, in one or more forms, for daily administration, based on a 70kg individual human.

### Mechanism of Action

While not Intending to be bound by any one theory as to the various therapeutic efficacies of the compositions described herein, it appears that the beneficial effect of the unique sterol/glucomannan combination, as described herein, rests on the complementary, yet different, mechanisms through which each component likely works.

It has been suggested in the literature that the presence of non- or semi-digestible carbohydrate in the GI tract possesses beneficial actions on the rate of release and uptake of glucose from digestible carbohydrate into the bloodstream. The blunting of the rate of the release of dietary carbohydrate is thought to suppress the speed of insulin release from the pancreas, and help in the overall process of blood glucose homeostasis. The suppression in blood of the rate of rise of insulin is also thought to depress the activation of whole body cholesterol biosynthesis, as it has been demonstrated that insulin potentiates the body's production of cholesterol. There may be some additional blocking of cholesterol intestinal absorption by glucomannan, as with other dietary fibres.

In comparison, it is assumed that phytosterols displace cholesterol from the micellar phase and thereby reduce its absorption or possibly compete with receptor and/or carrier sites in the cholesterol absorption process.

While sterols have been found to be non-toxic and perfectly safe for ingestion, even at high dosages, (with the exception to those patients having a rare condition called phytosterolemia) the same cannot be said for glucomannan, which may cause abdominal discomfort at high dosages. It is believed that on this complementary basis, the present authors have discovered a pairing between these two elements which capitalizes on the differing mechanisms of actions to achieve the desired therapeutic result, while at the same time minimizing the unpleasant side effects of bulk fibre administration.

### Methods of Use

The desired effects described herein may be achieved in a number of different ways. These compounds may be administered by any conventional means available for use in conjunction with pharmaceuticals, nutraceuticals, foods, beverages, and the like.

The amount of the compound which is required to achieve the desired effects will, of course, depend on a number of factors such as the particular compound chosen, the mode of administration and the condition of the patient.

The compounds of the present invention can be administered to a patient either by themselves, or in compositions where they are mixed with suitable carriers or excipients.

The compositions of the present invention may be manufactured in a manner that is itself known, e.g., by means of conventional mixing, dissolving, granulating, emulsifying, encapsulating, entrapping or lyophilizing processes.

In another form of the present invention, the compositions of the present invention may be administered through foods, beverages, nutritional supplements and nutraceuticals, including, without limitation, the following:
1) Dairy Products --such as cheeses, butter, milk and other dairy beverages, spreads and dairy mixes, ice cream and yoghurt;
2) Fat-Based Products--such as margarines, spreads, mayonnaise, shortenings, cooking and frying oils and dressings;
3) Cereal-Based Products--comprising grains (for example, bread and pastas) whether these goods are cooked, baked or otherwise processed;
4) Confectioneries--such as chocolate, candies, chewing gum, desserts, non-dairy toppings (for example Cool Whip™), sorbets, icings and other fillings;
5) Beverages-- whether alcoholic or non-alcoholic and including colas and other soft drinks, juice drinks, dietary supplement and meal replacement drinks such as those sold under the trade-marks Boost™ and Ensure™; and
6) Miscellaneous Products--including eggs and egg products, processed foods such as soups, pre-prepared pasta sauces, pre-formed meals and the like.

The compositions of the present invention may be incorporated directly and without further modification into the food, nutraceutical or beverage and the like by techniques such as mixing, infusion, injection, blending, dispersing, emulsifying, immersion, spraying and kneading. Alternatively, the compounds may be applied directly onto a food or into a beverage by the consumer prior to ingestion. These are simple and economical modes of delivery.

### EXAMPLES

The present invention is described by non-limiting example 2

### Example 1-Effect of Composition on Total and LDL Cholesterol-Human Study (Reference)

Summary and Results: The objective of the present study was to examine their effects on lipid profiles in mildly hypercholesterolemic subjects with and without type 2 diabetes. Thirteen type 2 diabetics (age = 56.31 ± 10.77 and BMI = 30.96 ± 3.37) and sixteen controls (age = 55.19 ± 6.46 and BMI =27.70 ± 4.32) were involved in a randomized crossover trial which contained 4 dietary periods of 21 d each. Each phase was separated by 28 days of wash-out period.

During each of the 4 treatment periods, subjects consumed either I) plant sterols (1.8g/d), 2) glucomannan (10g/d), 3) a combination of the two, or 4) a placebo in a random order.

Plant sterol and glucomannan were provided in a granola bar. Subjects maintained their usual diet during the study. Overall mean reduction of total cholesterol was -7.8% (plant sterols), - 10.4% (glucomannan), -13.1% (combination), and -2.4% (placebo). There was a significant difference between the combination and placebo treatment (p<0.05). Overall mean reduction of low-density lipoprotein (LDL) cholesterol levels for these conditions was -9.8% (PA), -14.3%, - 21.4%, and -2.4%, respectively. Plasma lathosterol levels, an index of cholesterol synthesis, were decreased (-23.3%, p<0.05) after the administration of the glucomannan/phytosterol composition compared to the placebo. Overall means of plasma campesterol levels, an index of cholesterol absorption, were lower (p<0.05) in the diabetic versus the non-diabetic group.

Combination treatment significantly decreased (p < 0.05) LDL cholesterol levels in both diabetic and control groups. The results clearly show that combination treatment using the composition of the present invention produces the greatest effect than either pant sterols or glucomannan alone. In conclusion, a composition of plant sterols and glucomannan is very effective in decreasing total cholesterol and LDL cholesterol levels in mildly hypercholesterolemic patients with or without type 2 diabetes.

### Study Details

The objectives were tested using a controlled cross-over clinical trial. The subjects consumed self-selecting meals. Dietitians instructed subjects to take fat (30%), carbohydrate (55%), protein (15%), nutritionally adequate diet during each period which was needed to address the objectives stated. The food intakes were assessed using the 24-hour food records for 3 days during each period. This experiment involved testing the effects of 4 separate diets on lipid metabolism in 15 healthy but hypercholesterolemic, marginally overweight subjects with type 2 diabetes and 15 lipid level- and weight-matched controls, using a randomized cross-over design where fat intakes were maintained constant across each of the two phases of the experiment:
i) Control.
ii) 1.8 g/d sterols, provided in margarine.
iii) 10g/d glucomannans, provided In a bar.
iv) 1.8 g/d sterols, and 10g/d glucomannans, provided in margarine and a bar.

Subject Selection: Healthy males and post-menopausal females, aged 40-80 yr, were be screened for determination of fasting circulating total cholesterol, triglyceride and glucose levels. Subjects accepted had plasma LDL cholesterol levels 3.4 - 5 mmol/l, triglyceride levels below 4 mmol/l and possessed hetero- or homozygousity for apolipoprotein E3. Diagnostic criteria for type 2 diabetes included fasting plasma glucose level of ≥ 7 mmol/l and plasma glucose level in a 2 hr sample after an standardized oral glucose tolerance test of ≥ 11.l mmol/l (75). Diabetic subjects possessed HbA_{1C} levels of 6-9%. Criteria for subjects of the control group included fasting and post-glucose tolerance test blood glucose levels of at least 20% below the above mentioned cut-off values. Type 2 diabetes and control subjects were matched for gender and body mass index (BMI) at entry into the study within the range of 23 to 30 kg/m².

Protocol and Diets: Subjects did undergo a randomized, cross-over trial containing 4 dietary periods of 21 d each separated by a washout interval of at least 4 wk, during each time, subjects consumed their regular diets without restriction. During each treatment period subjects consumed nutritionally adequate solid foods diets following dietician's Instruction. Of the 55% of energy provided as carbohydrate, complex carbohydrate made up at least 75%, with low glycemic index foods selected where possible. Subjects' dietary intakes were measured using 3 day food records in each period. Phytosterols (sitosterol [40%], campesterol [30%], dihydrobrassicasterol [20%], others [10%]) mixed in margarine were be provided subjects and they were Instructed to consume the margarine (ii) at the level to provide phytosterols at 1.8 g/day. Glucomannans were provided in a snack bar at a level of 10 g/d. Control margarine and bars were utilized in the phases where the treatments were not included. Subjects were advised to consume diets to maintain individual weight balance using a predictive equation based on each subject's weight, height, age, activity level, and gender. During the study, body weights were monitored weekly to examine possible effects of dietary intervention on body weight change.
Blood samples were collected at time zero and weekly over the experimental period for assessment of plasma lipoprotein levels of glucose, insulin, cholesterol in total, and LDL subfractions, Major phytosterol levels were also measured in diets and plasma using GLC (40).

The Friedewald equation was used to calculate LDL-C concentrations. Plant sterols sitosterol, campesterol, and dihydrobrassicasterol will be quantitated using GLC and a 5-alpha-cholestane internal standard

Determination of Cholesterol Absorption using the Dual Isotope Ratio Methodology: Free cholesterol extracted from RBCs was used to determine ¹³C- and D₇-cholesterol enrichment.

Determination of Cholesterol Biosynthesis: Cholesterol biosynthesis was determined as the rate of incorporation of deuterium from body water into RBC membrane free cholesterol over the period between 72 (day 20) and 96 (day 21) hr at the end of each treatment. Deuterium enrichment was measured in RBC free cholesterol and plasma water.

Statistical Power and Analyses: Group size (n=15 per group) was calculated to provide an 80% probability of detecting an anticipated difference in parameters tested of 20% using a within-group coefficient of variation (CV) of 15-20%. Synthesis measured in a group of mildly hypercholesterolemic subjects using deuterium incorporation in a previous experiment showed a CV of 18%.

### Example 2 Effects of Plant Sterols and Glucomannan on Cholesterol Absorption, Biosynthesis and Plasma Lipid Levels as well as Gycemic Control in Subjects with and without Type 2 Diabetes

Type 2 diabetic patients have a three-fold higher risk for developing CVD than non-diabetic subjects. Dyslipidemia often occurs in type 2 diabetics and cholesterol metabolism is altered in type 2 diabetes possibly due to decreased insulin sensitivity and disturbed glucose metabolism. Several studies report higher cholesterol synthesis and lower absorption rates in type 2 diabetics compared with non-diabetic subjects. Plant sterols lower circulating cholesterol levels by reducing intestinal cholesterol absorption. However, this cholesterol-lowering efficacy is often partially compensated by a simultaneous increase in cholesterol synthesis. As a soluble fiber, glucomannan lowers cholesterol levels by decreasing cholesterol biosynthesis rate by decreasing digestive rate and postprandial insulin secretion. In light of the fact that plant sterols and glucomannan decrease circulating cholesterol levels through different mechanisms, it is important to assess the hypocholesterolemic effect of this combination of plant sterols and glucomannan in type 2 diabetes and non-diabetes subjects.

The objectives were: i) To determine circulating lipid levels, plant sterol profile as well as glycemic control in response to supplementation of 1) plant sterols, 2) glucomannan, 3) a combination of each in type 2 diabetic and non-diabetic individuals.
ii) To determine whether the degree of response of circulating lipid and plant sterol levels, as well as, glycemic control are different between type 2 diabetic and non-diabetic individuals.

### Experimental Design and Methods

Sixteen non-diabetic (7 males and 9 females) and 13 type 2 diabetic (4 males and 9 females) volunteers with mild hypercholesterolemia were recruited through local newspapers. Subjects were aged between 38 to 74 years. Female subjects were postmenopausal. Subjects were asked to provide a medical history and to complete a physical examination prior to acceptance into the study. Fasting blood and urine samples were collected and sent to LDS Diagnostic Laboratories (Pointe Claire, QC, Canada) for biochemical, hematological, and urine analyses. Subjects with serum LDL cholesterol levels between 2.75 and 6.9 mmol/L and triglyceride levels below 4 mmol/L were selected for the study. For type 2 diabetic subjects fasting blood glucose levels ≥ 7 mmol/L and HbA1c levels between 6 to 9% were required. For non-diabetic subjects, the criteria of fasting blood glucose levels were less than 6.1 mmol/L. Subjects who had hypolipidemic or insulin therapy, chronic diseases such as gastrointestinal, renal, pulmonary, hepatic or biliary disease, and history of angina, cognitive heart failure, chronic use of laxatives were excluded from the study. Subjects were permitted to continue their medication of metformin, sulfonylurea, thyroid hormone, antihypertensive agents, and postmenopausal estrogens during the entire study.

### Experimental Design and Protocol

The study was a randomized crossover clinical trial involving 4 treatment phases of 21 days each and separated by a 4 week washout period between phases. Subjects were assigned into all 4 different treatments in a random order and consumed 1.8 g/day of plant sterols, 10 g/day of glucomannan, a combination of both, or none as a control. The plant sterol mixture contained 67.3% sitosterol, 10.8% sitostanol, 8.2% campesterol, 1.6% campestanol, and 12.1 % of others. Plant sterols and glucomannan were provided in the form of granola bars (Forbes Medi-tech Inc. Vancouver, BC, Canada). Nutritional compositions of the granola bars are presented in Table 1. Subjects were instructed to take 1 bar between meals as a snack, 3 times a day together with 250 mL of beverage. The bars were provided to subjects each week (day 0, 8, and 15) at the Mary Emily Clinical Nutrition Research Unit of McGill University (Montreal, QC, Canada). Compliance was assessed by weighing the amount of returned granola bars weekly. Physical examinations were performed at the beginning (day 0) and the end (day 21) of each phase by the study physician to ensure the health status of all participants. During the study subjects were asked to maintain their dietary habit. Body weight was measured weekly (day 0, 8, 15, and 21). Side effects and dietary changes during the study period were monitored by questionnaire at the beginning (day 0) and the end (day 21) of each treatment phase.

Fasting blood samples were taken at day 0, day 8, 15, and 21, during each phase. Plasma and red blood cell were separated by centrifugation for 15 min at 1500 rpm within 30 min of phlebotomy and were stored at -20 °C until analysis. Plant sterols, insulin, and fructosamine levels were measured at the beginning (day 0) and end of each phase (day 21). On the last day of each phase (day 21), after fasting blood sample collection, 2-hour oral glucose tolerance test was conducted. A liquid containing 50g glucose was given orally to each subject and finger prick blood samples were taken every half an hour for 2 hours. The palatability of the treatment granola bars was evaluated by questionnaire in the end of each treatment phase.

### Determination of plasma lipid concentrations

Plasma total cholesterol, LDL cholesterol, HDL cholesterol and triglyceride concentrations were determined enzymatically using a Hitachi/991 chemistry analyzer (Roche Diagnostic Inc. Indiana IN) at the Lachine Hospital (Montreal, QC, Canada). Plasma LDL cholesterol levels were calculaled using the Friedewald equation.

### Determination of plasma plant sterol concentrations

Plasma plant sterol concentrations were measured in duplicate by gas-liquid chromatography as commonly used in this field. Briefly, 1.0 mL of plasma was saponified with methanolic KOH at 100 °C for 2 h. The non-saponified lipids were extracted with petroleum ether. An internal standard of 5-α-cholestane was added at the beginning of lipid extraction. The lipid extracts were analyzed by a gas-liquid chromatography equipped with a flame ionization detector (HP 5890 series II; Hewlett Packard, Palo Alto CA) using a 30 m capillary column with i.d. of 0.25 mm (SAC-5; Supelco, Bellefonte PA). Detector and injector temperatures were set 310 and 300 °C, respectively. Samples were run isothermally at 285 °C. Plant sterol peaks were identified by comparison with authenticated standards (Sigma-Aldrich Canada Ltd., Oakville ON, Canada).

Determination of plasma insulin, serum fructosamine and blood glucose concentrations Plasma insulin concentrations were determined in duplicate, using commercially available radioimmunoassay kits (ICN Pharmaceutical, Inc., Costa Mesa, CA) utilizing ¹²⁵I as a tracer. Radioactivity was determined by gamma counter (LKB Wallac, 1282 compugamma CS, Fisher Scientific, Montreal, QC, Canada) and expressed as count per min (CPM). Plasma insulin concentrations were quantified in reference to a standard curve. Serum fructosamine concentrations were determined by LDS Diagnostic Laboratories (Pointe Claire, QC, Canada). Blood glucose levels were measured using a portable glucometer, Glucometer Elite^{®} XL (Bayer Inc., Toronto, ON, Canada).

### Results

A total of 16 type 2 diabetic and 18 non-diabetic individuals participated the study, with 13 and 16 successfully completing all treatments, respectively. Two subjects (1 non-diabetic and 1 type 2 diabetic subject) withdrew their participation after glucomannan supplementation, due to gastric discomfort. Results of 3 subjects (1 non-diabetic and 2 type 2 diabetic subjects) were excluded from the statistical analysis due to low compliance (less than 50%). The baseline characteristics of subjects are presented in **Table 2.**

Body mass index, plasma triglyceride, and all of the diabetic indicators including glucose, insulin, HbA1c and fructosamine were higher (p<0.05) in type 2 diabetic group than non-diabetic group. However, plasma LDL cholesterol levels were lower (p<0.01) in diabetic subjects compared with non-diabetic individuals.

The consumption of test granola bars was 98.8%, 99.4%, 98.4%, and 98.8% in control, plant sterols, glucomannan, and combination treatment, respectively (**Table 3**). There was no difference between the two groups of subjects or between dietary treatments in the consumption of granola bars.

### Palatability of treatment granola bars

The palatability of treatment granola bars was evaluated from 0 to 10. Higher score showed the greater palatability. As shown in **Table 4**, the palatability of granola bar was 7.16 ± 0.39, 7.63 ± 0.38, 6.36 ± 0.48, and 6.04 ± 0.36 in control, plant sterols, glucomannan, and combination treatments, respectively. Plant sterol-containing granola bars showed higher (p<0.05) palatability than glucomannan-containing bars or the bars containing both glucomannan and plant sterols. Type 2 diabetics showed higher acceptance for the tested granola bars than non-diabetics.

### Effects of dietary treatments on body weight

The mean body weight changes from baseline were -0.22 ± 0.32, -0.10 ± 0.18, -0.05 ± 0.18, and -0.70 ± 0.24 kg in control, plant sterols, glucomannan, and the combination treatments, respectively (**Table 5**). During the combination treatment, type 2 diabetics decreased (p<0.05) their body weight (-1.34 ± 0.24 kg), while non-diabetics maintained their body weight (-0.29 ± 0.33 kg). In other treatment periods, no significant changes in body weight were observed as compared to the baselines and between the two groups of subjects (Table 5.). No significant dietary changes were reported during the entire study period.

### Effects of dietary treatments on plasma lipids

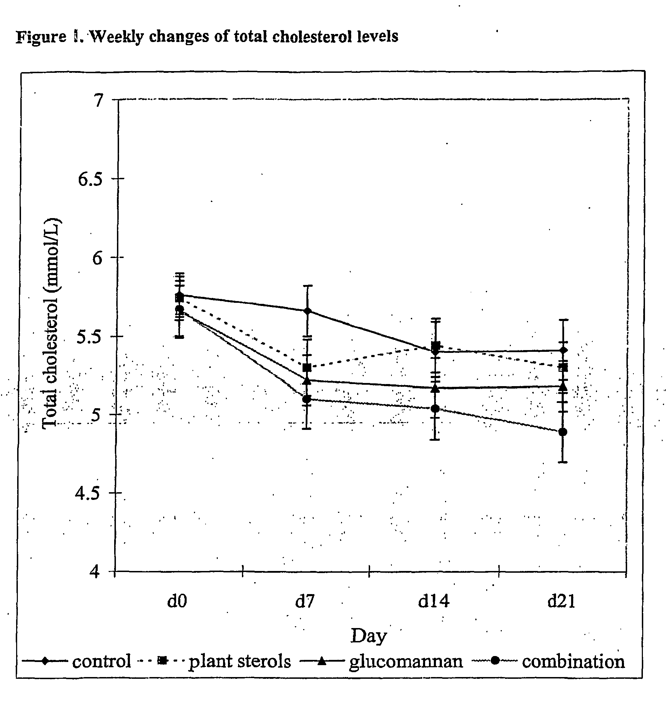

Plasma lipid levels of non-diabetic and type 2 diabetic groups during the treatment periods were shown in **Table 6**. Supplementation of glucomannan combined with plant sterols reduced (p<0.05) plasma total cholesterol levels (4.77 ± 0.20 mmol/L) compared to control (5.38 ± 0.18 mmol/L). The percentage change of total cholesterol levels from baseline was lower (p<0.05) in glucomannan (-12.40 ± 2.32%) and combination (-14.80 ± 3.86%) treatments compared to control (-3.20 ± 1.49%). There was no difference between groups of subjects in response to the treatments. The weekly changes of plasma total cholesterol levels were shown in **Figure 1****.** After 1 week, plasma total cholesterol levels were decreased (p<0.05) in plant sterol, glucomannan, and combination treatments compared to control. Although the degree of reduction of total cholesterol was gradually diminished after 2 weeks of plant sterol and glucomannan treatments, the decrease of total cholesterol concentrations was still observed with the combination treatment

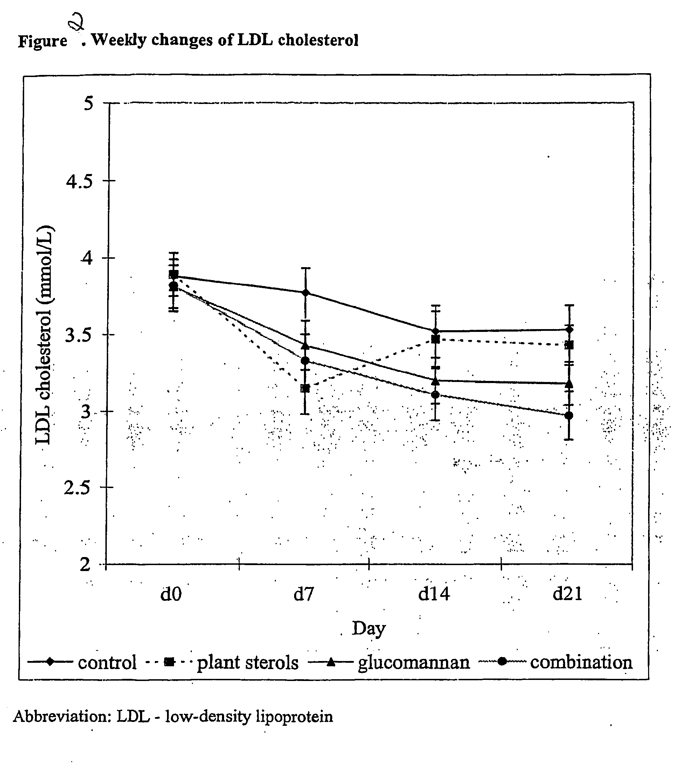

On day 0, plasma LDL cholesterol levels were lower (p<0.05) in the type 2 diabetic group (4.06 ± 0.20 mmol/L) compared with the non-diabetic group (3.47 ±0.17 mmol/L). After 21 days of supplementation, glucomannan (3.18 ± 0.14 mmol/L) and the combination of glucomannan and plant sterols (3.00 ± 0.16 mmol/L) decreased (p<0.05) LDL cholesterol levels compared to control (3.53 ± 0.16 mmol/L). Plasma LDL cholesterol levels were also lower (p<0.05) after combination treatment (3.00 ± 0.16 mmol/L) compared with plant sterol treatment (3.43 ± 0.13 mmol/L). The weekly changes of LDL cholesterol levels during the study were shown in Figure 2. LDL cholesterol was reduced (p<0.05) after 2 weeks of the combination treatment. The percent change of LDL cholesterol levels from day 1 to day 21 reduced (p<0.05) by combination treatment (-21.27± 3.76%) relative to control (-4.51 ± 3.02%). There was no difference between two groups of subjects in the changes of LDL cholesterol levels after each dietary treatment

Plasma HDL cholesterol levels did not differ among the treatment period. There was also no difference in the changes of HDL levels between two subject groups.The ratio of plasma LDL cholesterol to HDL cholesterol was lower (p<0.05) after plant sterol (3.41 ± 0.19) and glucomannan treatments (3.27 ± 0.17) compared to control (3.68 ± 0.19). Furthermore, plasma LDL cholesterol to HDL cholesterol ratio in the combination treatment (3.11 ± 0.17) was lower (p<0.05) than both the control (3.68 ± 0.19) and plant sterol (3.41 ± 0.19) treatments. No differences were observed between the two groups in LDL cholesterol to HDL cholesterol ratio.

Total cholesterol to HDL cholesterol ratio was lower (p<0.05) after 21 days on the combination treatment (5.03 ± 0.22) than control (5.65 ± 0.25) and plant sterol treatments (5.36 ± 0.28). Total cholesterol to HDL cholesterol ratio was lower (p<0.05) in subjects taking glucomannan (5.20 ± 0.24) compared to control treatment (5.65 ± 0.25).

Triglyceride levels were not affected by dietary treatments. However, the plasma triglyceride levels were consistently higher (p<0.05) in diabetic group than in non-diabetic group during the entire study period.

### Effects of dietary treatments on plasma plant sterols

Plasma plant sterols in type 2 diabetic and non-diabetic groups were summarized in Table 7. The overall mean of plasma campesterol levels was lower (p<0.05) in the diabetic group (5.77 ± 0.42 µmol/L) than in the non-diabetic group (7.59 ± 0.38 µmol/L). There was no difference among treatments.

There was no difference in the plasma lathosterol levels between two groups in response to the treatments. However, the reductions after 21 days were greater (p<0.05) with the combination treatment (-1.25 ± 0.36 µmol/L) compared with plant sterol treatment (0.17 ± 0.40 µmol/L). On day 21, β-sitosterol levels and the ratio of β-sitosterol to total cholesterol were higher (p<0.05) in plant sterol treatment (5.50 ± 0.76 µmol/L and 1.11 ± 0.18 mmol/mol) compared to the glucomannan treatment (3.81 ± 0.45 µmol/L and 0.75 ± 0.45 mmol/mol). Although it was not significant, β-sitosterol levels and the ratio of β-sitosterol to total cholesterol in the combination treatment (4.72 ± 0.51 µmol/L and 1.03 ± 0.12mmol/mol) were higher than the control (4.53 ± 0.47µmol/L and 0.88 ± 0.10mmol/mol) and glucomannan treatments. Plasma β-sitosterol levels were not different between type 2 diabetic and non-diabetic groups after each treatment.

### Effects of dietary treatments on insulin, fructosamine, and 2hr oral glucose tolerance test

The plasma insulin levels were higher (p<0.01) in the diabetic group (39.16 ± 2.24 µIU/mL) than in the non-diabetic group (21.01 ± 1.00 µIU/mL)(**Table 8**). The ratio of plasma insulin levels relative to control treatment was lower (p<0.05) in the glucomannan (-79.7 ± 25.08%) than the plant sterol (1.71 ± 6.16%) and combination treatments (-5.64 ± 4.81%). Serum fructosamine levels were higher (p<0.01) in diabetic subjects (3.95 ± 0.07 umol/g) than non-diabetic subjects (3.40 ± 0.03 umol/g) (**Table 9**). However, dietary treatments did not affect serum fructosamine concentrations.

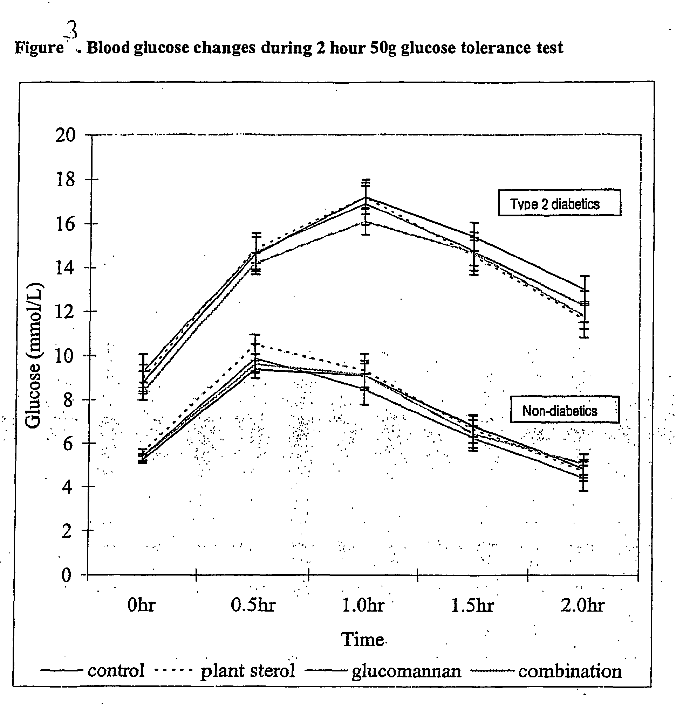

The blood glucose levels were higher in type 2 diabetics than in non-diabetics during 50g 2 hour oral glucose test (**Figure 3**). The dietary treatments did not affect glucose tolerance.

### Discussion

The main finding of the present study is that the combination of plant sterols and glucomannan had a greater cholesterol-lowering effect than plant sterols and glucomannan alone in both type 2 diabetic and non-diabetic mild hypercholesterolemic individuals. The present study showed a decrease of plasma LDL cholesterol levels by 10.6%, 14.5%, and 21.3% in plant sterols, glucomannan, and combination treatments, respectively. These results indicate that plant sterols and glucomannan have at least an additive effect in lowering cholesterol concentration. Plant sterols reduce cholesterol incorporation into micelle and thus suppress dietary and biliary cholesterol absorption in the intestine.. On the other hand, glucomannan suppresses the postprandial insulin peak by delaying the absorption of nutrients in the small intestine. The reduced postprandial insulin concentrations decrease cholesterol biosynthesis possibly by increasing HMG-CoA activity. Thus, resulting in the greater hypocholesterolemic effects of the combination of plant sterols and glucomannan compared to plant sterols and glucomannan alone.

In the current study, plasma lathosterol level (0.17 ± 0.40 µmol/L) was increased from baseline with plant sterol treatment, however, it was decreased after consumption of the combination treatment of plant sterols and glucomannan (-1.25 ± 0.36 µmol/L), suggesting that glucomannan suppressed the increase of cholesterol synthesis caused by plant sterols. It has been observed that altered cholesterol metabolism exists in type 2 diabetic individuals. Compared to non-diabetic subjects, type 2 diabetics have higher hepatic cholesterol synthesis and lower intestinal absorption. Serum plant sterol levels are indicators of cholesterol absorption rate when subjects are taking diets without plant sterols or having approximately equal amount of plant sterols. In fact, it has been reported that serum plant sterol concentrations are lower in type 2 diabetics than in non-diabetics, supporting the fact that cholesterol absorption is decreased in this population. Plasma campesterol levels in the diabetic group (5.77 ± 0.42 µmol/L) were lower (p<0.05) than in non-diabetic group (7.60 ± 0.38 µmol/L), which support previous observations. It is postulated that lower insulin sensitivity in diabetic individuals is related to the alteration of cholesterol metabolism. In the current study, lower (p<0.05) plasma campesterol levels were observed in diabetic subjects (5.77 ± 0.42 µmol/L) compared to non-diabetic subjects (7.59 ± 0.38 µmol/L), indicating that cholesterol homeostasis was indeed altered in these subjects.

In the present study, dietary supplementation of glucomannan did not improve the glycemic indexes such as fasting insulin and fructosamine levels. However, hypoglycemic effects of glucomannan have been demonstrated in previous studies in both healthy and type 2 diabetic individuals. Hopman *et al.* (1988) reported that dietary supplementation of 2.6 g and 5.2 g of glucomannan decreased the postprandial rise in plasma insulin in patients with previous gastric surgery. These results were supported by Scalfi *et al.* (1987) who found similar results at a dose of 6 g/day glucomannan. After 30 days and 65 days of glucomannan supplementation fasting blood glucose levels and the 2 hr-postprandial blood glucose levels were significantly reduced (Huang, 1990). The discrepancy between the present and previous studies may be due to the absence of dietary control.

In summary, results of the present study show that both plant sterols and glucomannan lower circulating cholesterol levels, however, the combination of plant sterols and glucomannan had an additive or greater effect and induced a greater reduction in total and LDL cholesterol levels than plant sterols and glucomannan alone. Contrary to what was expected, no changes in glycemic control were observed by supplementations of plant sterols and/or glucomannan treatments. Plasma lathosterol levels were increased after ingestion of plant sterols, however, by adding glucomannan to plant sterols, these changes were reversed. Thus, the combination of plant sterols and glucomannan can be considered to exist as an alternative to pharmacological approaches to lower LDL cholesterol levels in type 2 diabetic and non-diabetic hypercholesterolemic individuals.

**Table 1. Nutritional composition of granola bars**

| Nutrients (per serving) | Control | Plant sterols | Glucomannan | Combination |
|---|---|---|---|---|
| Total energy (kcal) | 120.9 | 129.3 | 109.1 | 118.4 |
| Carbohydrate (g) | 20.2 | 19.8 | 17.7 | 17.1 |
| Protein (g) | 2.5 | 2.4 | 1.9 | 2.0 |
| Fat (g) | 3.4 | 4.5 | 3.4 | 4.7 |
| Saturated fat (g) | 0.6 | 0.4 | 0.7 | 0.4 |
| Sodium (mg) | 93.5 | 63.4 | 83.3 | 52.8 |
| Fiber (g) | 1.5 | 1.5 | 4.5 | 4.5 |
| Vitamin A (IU) | 258.4 | 145.9 | 232.9 | 121.0 |
| Vitamin C (mg) | 1.6 | 1.6 | 1.2 | 1.3 |
| Fe (mg) | 0.8 | 0.8 | 0.7 | 0.7 |
| Calcium (mg) | 14.6 | 13.4 | 12.9 | 11.7 |

**Table 2. Baseline characteristics of subjects¹**

| Variables | Non-diabetics (n=16) | Type 2 diabetic (n=13) |
|---|---|---|
| Male/Female | 7/9 | 4/9 |
| Ages (y) | 55.19 ± 1.67 | 56.81 ± 3.11 |
| Body mass index (kg/m²) | 27.7 ± 1.12 | 30. 96 ± 0.97* |
| Lipids (mmol/L) | | |
| Total cholesterol | 6.05 ± 0.21 | 5.53 ± 0.21 |
| LDL cholesterol² | 4.29 ± 0.19 | 3.53 ± 0.17** |
| HDL cholesterol³ | 1.11 ± 0.09 | 0.92 ± 0.05 |
| Triglycerides | 1.43 ± 0.13 | 2.69 ± 0.44** |
| Glucose (mmol/L) | 5.11 ± 0.12 | 8.56 ± 0.40*** |
| Insulin (µIU/mL) | 21.03 ± 2.04 | 35.96 ± 4.49** |
| HbA1c | 0.06 ± 0.001 | 0.07 ± 0.003*** |
| Fructosamine (umol/g) | 3.41 ± 0.08 | 4.09 ± 0.16*** |

| | | |
|---|---|---|
| ¹Values are expressed as mean ± SE. * P<0.05, ** P<0.01, *** P<0.001 significant difference from non diabetic group. ²Low-density lipoprotein cholesterol ³High-density lipoprotein cholesterol | | |

**Table 3. Average consumption of granola bars¹**

| Consumption (%) | Control | Plant sterols | Glucomannan | Combination |
|---|---|---|---|---|
| Non-diabetics | 98.8 ± 0.006 | 99.4 ± 0.003 | 98.5 ± 0.008 | 99.0 ± 0.008 |
| Type 2 diabetics | 97.6 ± 0.008 | 98.9 ± 0.006 | 98.4 ± 0.009 | 98.5 ± 0.013 |
| All | 98.3 ± 0.005 | 99.2 ± 0.003 | 98.4 ± 0.006 | 98.8 ± 0.007 |

| | | | | |
|---|---|---|---|---|
| *¹*Values are expressed as mean ± SE. Percent change is based on individual data. Percent change relative to control is based on the mean of day 21. | | | | |

**Table 4. Palatability of granola bars¹**

| Palatability | Control | Plant sterols | Glucomannan | Combination |
|---|---|---|---|---|
| Non-diabetics | 6.31 ± 0.49 | 7.00 ± 0.51 | 5.69 ± 0.61 | 6.06 ± 0.37 |
| Type 2 diabetics | 8.29 ± 0.49** | 8.46 ± 0.51 | 7.25 ± 0.70 | 6.00 ± 0.71 |
| All | 7.16 ± 0.39*^{ab}* | 7.63 ± 0.38*^{a}* | 6.36 ± 0.48*^{b}* | 6.04 ± 0.36*^{b}* |

| | | | | |
|---|---|---|---|---|
| *¹*Values are expressed as mean ± SE. Values carrying different superscript letters indicate a significant difference among treatments (p<0.05). **P<0.01; significant difference from non diabetic group. | | | | |

**Table 5. Body weight change at day 0 and day 21 of each treatment period¹**

| Body weight (kg) | | Control | Plant sterols | Glucomannan | Combination |
|---|---|---|---|---|---|
| *Non-diabetics* | | | | | |
| | Day 0 | 75.28 ± 3.92 | 75.02 ± 3.64 | 74.29 ± 3.64 | 75.02 ± 3.81 |
| | Day 21 | 75.00 ± 3.66 | 75.15 ± 3.66 | 74.34 ± 3.59 | 74.73 ± 3.82 |
| | Difference² | -0.28 ± 0.44 | 0.13 ± 0.20 | 0.50 ± 0.26 | -0.29 ± 0.33 |
| | % Change | -0.17 ± 0.50 | 0.17 ± 0.28 | 0.13 ± 0.33 | -0.41 ± 0.39 |
| | | | | | |
| *Type 2 diabetics* | | | | | |
| | Day 0 | 80.81 ± 3.05 | 81.12 ± 2.96 | 81.54 ± 2.89 | 81.76 ± 3.00 |
| | Day 21 | 81.69 ± 2.92 | 81.64 ± 2.77 | 82.11 ± 2.81 | 81.68 ± 2.87 |
| | Difference² | -0.13 ± 0.46 | -0.46 ± 0.30 | -0.21 ± 0.24 | -1.34 ± 0.24* |
| | % Change | -0.14 ± 0.55 | -0.48 ± 0.35 | -0.26 ± 0.27 | -1.61 ± 0.27* |
| | | | | | |
| *All* | | | | | |
| | Day 0 | 77.53 ± 2.65 | 77.50 ± 2.50 | 77.24 ± 2.51 | 77.76 ± 2.61 |
| | Day 21 | 77.72 ± 2.51 | 77.79 ± 2.48 | 77.51 ± 2.49 | 77.56 ± 2.59 |
| | Difference² | -0.22 ± 0.32 | -0.10 ± 0.18 | -0.05 ± 0.18 | -0.70 ± 0.24 |
| | % Change | -0.16 ± 0.36 | -0.08 ± 0.22 | -0.02 ± 0.23 | -0.87 ± 0.28 |

| | | | | | |
|---|---|---|---|---|---|
| *¹*Values are expressed as mean ± SE. Percent change is based on individual data. Percent change relative to control is based on the mean of day 21. *P<0.05; significant difference from non diabetic group. *²*Difference between day 1 and day 21. | | | | | |

**Table 6. Plasma lipid levels at day 0 and day 21 of each treatment period¹**

| Lipid (mmo/L) | | Control | Plant sterols | Glucomannan | Combination |
|---|---|---|---|---|---|
| Total cholesterol | | | | | |
| *Non-diabetics* | | | | | |
| | Day 0 | 5.88 ± 0.21 | 5.90 ± 0.21 | 5.86 ± 0.23 | 5.81 ± 0.29 |
| | Day 21 | 5.52 ± 0.28 | 5.46 ± 0.19 | 5.07 ± 0.27 | 4.77 ± 0.35 |
| | % Change | -1.82 ± 1.95 | -7.31 ± 1.75 | -13.92 ± 3.23 | -16.79 ± 6.67 |
| | % Relative to C*²* | | -4.36 ± 1.84 | -12.57 ± 2.85 | -18.23 ± 5.10 |
| | | | | | |
| *Type 2 diabetics* | | | | | |
| | Day 0 | 5.61 ± 0.18 | 5.54 ± 0.19 | 5.42 ± 0.19 | 5.49 ± 0.20 |
| | Day 21 | 5.21 ± 0.20 | 5.06 ± 0.23 | 4.84 ± 0.16 | 4.77 ± 0.15 |
| | % Change | -4.93 ± 2.30 | -8.41 ± 2.12 | -10.50 ± 3.38 | -12.3 ± 2.76 |
| | % Relative to C*²* | | -2.21 ± 3.96 | -5.95 ± 4.11 | -7.71 ± 2.81 |
| | | | | | |
| *All* | | | | | |
| | Day 0 | 5.76 ± 0.14 | 5.74 ± 0.14 | 5.66 ± 0.16 | 5.67 ± 0.18 |
| | Day 21 | 5.38 ± 0.18*^{a}* | 5.28 ± 0.15*^{ab}* | 4.97 ± 0.16*^{ab}* | 4.77 ± 0.20*^{b}* |
| | % Change | -3.20 ± 1.49*^{a}* | -7.80 ± 1.33*^{ab}* | -12.40 ± 2.32*^{b}* | -14.80 ± 3.86*^{b}* |
| | % Relative to C*²* | | -3.36 ± 2.05*^{a}* | -9.50 ± 2.48*^{ab}* | -13.35 ± 3.14*^{b}* |
| | | | | | |
| LDL cholesterol*³* | | | | | |
| *Non-diabetics* | | | | | |
| | Day 0 | 4.08 ± 0.20 | 4.10 ± 0.21 | 4.06 ± 0.20 | 4.05 ± 0.24 |
| | Day 21 | 3.77 ± 0.24 | 3.66 ± 0.15 | 3.42 ± 0.23 | 3.11 ± 0.26 |
| | % Change | -3.07 ± 3.31 | -10.67 ± 2.14 | -15.78 ± 4.04 | -26.38 ± 6.00 |
| | % Relative to C*²* | | -5.42 ± 2.28 | -14.33 ± 3.44 | -22.73 ± 5.42 |
| | | | | | |
| *Type 2 diabetics* | | | | | |
| | Day 0 | 3.61 ± 0.22 | 3.60 ± 0.15 | 3.47 ± 0.17* | 3.51 ± 0.18 |
| | Day 21 | 3.26 ± 0.17 | 3.17 ± 0.20 | 2.90 ± 0.12 | 2.81 ± 0.15 |
| | % Change | -6.21 ± 5.44 | -10.45 ± 4.11 | -12.92 ± 4.06 | -15.24 ± 3.87 |
| | % Relative to C*²* | | -1.56 ± 5.40 | -8.42 ± 5.63 | -12.84 ± 3.71 |
| | | | | | |
| *All* | | | | | |
| | Day 0 | 3.88 ± 0.15 | 3.89 ± 0.14 | 3.81 ± 0.14 | 3.82 ± 0.17 |
| | Day 21 | 3.53 ± 0.16*^{a}* | 3.43 ± 0.13*^{ab}* | 3.18 ± 0.14*^{bc}* | 3.00 ± 0.16*^{c}* |
| | % Change | -4.51 ± 3.02*^{a}* | -10.57 ± 2.16*^{ab}* | -14.47 ± 2.83*^{ab}* | -21.27 ± 3.76*^{b}* |
| | % Relative to C*²* | | -3.56 ± 2.82*^{a}* | -11.59 ± 3.19*^{b}* | -18.14 ± 3.45*^{b}* |
| | | | | | |
| Triglyceride | | | | | |
| *Non-diabetics* | | | | | |
| | Day 0 | 1.42 ± 0.15 | 1.42 ± 0.14 | 1.35 ± 0.13 | 1.29 ± 0.12 |
| | Day 21 | 1.41 ± 0.16 | 1.40 ± 0.15 | 1.19 ± 0.15 | 1.29 ± 0.19 |
| | % Change | -1.80 ± 6.91 | 5.44 ± 8.77 | -14.28 ± 6.37 | -6.47 ± 6.65 |
| | % Relative to C*²* | | 5.57 ± 8.64 | -11.01 ± 8.47 | -5.32 ± 11.94 |
| | | | | | |
| *Type 2 diabetics* | | | | | |
| | Day 0 | 2.32 ± 0.29** | 2.16 ± 0.23** | 2.03 ± 0.19** | 2.20 ± 0.19*** |
| | Day 21 | 2.22 ± 0.24** | 2.00 ± 0.23* | 2.15 ± 0.25** | 2.18 ± 0.22** |
| | % Change | 0.95 ± 13.14 | -10.54 ± 7.63 | -0.25 ± 10.23 | -3.22 ± 7.47 |
| | % Relative to C*²* | | -3.00 ± 10.56 | 5.07 ± 11.41 | 6.55 ± 10.27 |
| | | | | | |
| *All* | | | | | |
| | Day 0 | 1.82 ± 0.17 | 1.75 ± 0.14 | 1.65 ± 0.13 | 1.70 ± 0.14 |
| | Day 21 | 1.78 ± 0.16 | 1.68 ± 0.14 | 1.63 ± 0.17 | 1.71 ± 0.16 |
| | % Change | -0.48 ± 7.13 | -2.26 ± 5.94 | -7.53 ± 5.97 | -4.90 ± 4.90 |
| | % Relative to C*²* | | 1.64 ± 6.66 | -3.55 ± 7.01 | 0.19 ± 7.91 |
| | | | | | |
| HDL cholesterol*⁴* | | | | | |
| *Non-diabetics* | | | | | |
| | Day 0 | 1.11 ± 0.09 | 1.13 ± 0.09 | 1.07 ± 0.08 | 1.09 ± 0.09 |
| | Day 21 | 1.03 ± 0.07 | 1.11 ± 0.09 | 1.05 ± 0.09 | 0.99 ± 0.08 |
| | % Change | -0.79 ± 2.62 | -2.95 ± 2.33 | -3.63 ± 3.02 | -5.20 ± 7.06 |
| | % Relative to C*²* | | 8.89 ± 8.99 | 3.63 ± 9.39 | 0.11 ± 9.92 |
| | | | | | |
| *Type 2 diabetics* | | | | | |
| | Day 0 | 0.97 ± 0.05 | 0.97 ± 0.06 | 0.99 ± 0.06 | 0.96 ± 0.04 |
| | Day 21 | 0.94 ± 0.06 | 0.99 ± 0.06 | 0.96 ± 0.06 | 0.96 ± 0.05 |
| | % Change | -3.28 ± 3.72 | 2.81 ± 2.40 | -2.56 ± 2.69 | 0.55 ± 1.65 |
| | % Relative to C*²* | | 6.00 ± 3.51 | 2.97 ± 3.82 | 4.30 ± 3.42 |
| | | | | | |
| *All* | | | | | |
| | Day 0 | 1.05 ± 0.05 | 1.05 ± 0.06 | 1.03 ± 0.05 | 1.03 ± 0.55 |
| | Day 21 | 0.99 ± 0.04 | 1.05 ± 0.06 | 1.01 ± 0.05 | 0.98 ± 0.05 |
| | % Change | -1.94 ± 2.20 | -0.27 ± 1.73 | -3.34 ± 2.01 | -2.53 ± 3.84 |
| | % Relative to C*²* | | 7.59 ± 5.13 | 3.33 ± 5.37 | 1.99 ± 5.61 |
| | | | | | |
| LDL:HDL ratio*^{3,4}* | | | | | |
| *Non-diabetics* | | | | | |
| | Day 0 | 4.03 ± 0.36 | 3.97 ± 0.37 | 4.21 ± 0.36 | 3.69 ± 0.41 |
| | Day 21 | 3.78 ± 0.32 | 3.51 ± 0.31 | 3.40 ± 0.28 | 3.24 ± 0.28 |
| | % Change | -1.14 ± 1.38 | 0.42 ± 0.85 | 0.61 ± 1.76 | -3.41 ± 2.52 |
| | % Relative to C*²* | | -6.79 ± 3.33 | -8.56 ± 3.60 | -14.03 ± 2.92 |
| | | | | | |
| *Type 2 diabetics* | | | | | |
| | Day 0 | 3.65 ± 0.25 | 3.72 ± 0.20 | 3.50 ± 0.26 | 3.67 ± 0.23 |
| | Day 21 | 3.56 ± 0.20 | 3.30 ± 0.23 | 3.12 ± 0.18 | 3.00 ± 0.16 |
| | % Change | 2.38 ± 1.42 | -0.71 ± 1.69 | 2.98 ± 1.20 | 5.70 ± 3.87 |
| | % Relative to C*²* | | -7.12 ± 4.13 | -11.69 ± 2.92 | -16.61 ± 1.76 |
| | | | | | |
| *All* | | | | | |
| | Day 0 | 3.87 ± 0.24 | 3.87 ± 0.23 | 3.92 ± 0.25 | 3.68 ± 0.26 |
| | Day 21 | 3.68 ± 0.19*^{a}* | 3.41 ± 0.19*^{b}* | 3.27 ± 0.17*^{bc}* | 3.11 ± 0.17*^{c}* |
| | % Change | 0.30 ± 1.05 | -0.43 ± 0.84 | 1.58 ± 1.16 | 0.32 ± 1.16 |
| | % Relative to C*²* | | -6.94 ± 2.57*^{a}* | -10.01 ± 2.33*^{a}* | - 15.23 ± 1.75*^{b}* |
| | | | | | |
| TC:HDL ratio^{4*,5*} | | | | | |
| *Non-diabetics* | | | | | |
| | Day 0 | 5.71±0.42 | 5.71±0.46 | 5.87 ± 0.43 | 5.72 ± 0.43 |
| | Day 21 | 5.60 ± 0.40 | 5.42 ± 0.47 | 5.20 ± 0.41 | 5.02 ± 0.37 |
| | % Change | -0.95 ± 0.74 | 0.22 ± 0.67 | 0.70 ± 1.37 | -2.15 ± 1.88 |
| | % Relative to C*²* | | -3.59 ± 3.30 | -6.61 ± 3.43 | -10.25 ± 2.30 |
| | | | | | |
| *Type 2 diabetics* | | | | | |
| | Day 0 | 5.91 ± 0.30 | 5.92 ± 0.29 | 5.70 ± 0.36 | 5.84 ± 0.27 |
| | Day 21 | 5.71 ± 0.28 | 5.28 ± 0.28 | 5.20 ± 0.24 | 5.04 ± 0.21 |
| | % Change | 1.75 ± 1.05* | -0.76 ± 0.81 | 1.38 ± 0.88 | 2.91 ± 2.08 |
| | % Relative to C*²* | | -7.47 ± 2.78 | -8.43 ± 2.67 | -11.03 ± 2.51 |
| | | | | | |
| *All* | | | | | |
| | Day 0 | 5.80 ± 0.26 | 5.81 ± 0.28 | 5.80 ± 0.28 | 5.77 ± 0.26 |
| | Day 21 | 5.65 ± 0.25*^{a}* | 5.36 ± 0.28*^{ab}* | 5.20 ± 0.24*^{bc}* | 5.03 ± 0.22*^{c}* |
| | % Change | 0.24 ± 0.67 | -0.21 ± 0.52 | 1.00 ± 0.85 | 0.08 ± 1.46 |
| | % Relative to C*²* | | -5.33 ± 2.20*^{a}* | -7.43 ± 2.21*^{ab}* | -10.60 ± 1.67*^{b}* |

| | | | | | |
|---|---|---|---|---|---|
| *¹*Values are expressed as mean ± SE. Values carrying different superscript letters indicate a significant difference among treatments (p<0.05). Percent change is based on individual data. Percent change relative to control is based on the mean of day 21. *P<0.05; **P<0.01; ***P<0.001; significant difference from non diabetic group. *²*Control treatment *³*Low-density lipoprotein cholesterol *⁴*High-density lipoprotein cholesterol *⁵*Total cholesterol | | | | | |

**Table 7. Plasma plant sterol levels at day 0 and day 21 of each treatment period ¹**

| Phytosterol (µmol/L) | | Control | Plant sterols | Glucomannan | Combination |
|---|---|---|---|---|---|
| Campesterol | | | | | |
| *Non-diabetics* | | | | | |
| | Day 21 | 7.83 ± 0.83 | 7.35 ± 0.71 | 7.83 ± 0.91 | 7.34 ± 0.62 |
| | Difference² | 0.65 ± 0.84 | -0.86 ± 0.87 | 0.79 ± 0.72 | -0.33 ± 0.85 |
| | mmol/mol³ | 1.48 ± 0.18 | 1.45 ± 0.21 | 1.47 ± 0.17 | 1.66 ± 0.21 |
| | | | | | |
| *Type 2 diabetics* | | | | | |
| | Day 21 | 5.59 ± 0.90 | 6.46 ± 0.91 | 5.83 ± 0.85 | 5.19 ± 0.85* |
| | Difference² | -0.51 ± 0.49 | 0.20 ± 0.89 | -0.20 ± 0.90 | -0.95 ± 0.96 |
| | mmol/mol³ | 1.16 ± 0.24 | 1.29 ± 0.8 | 1.18 ± 0.16 | 1.08 ± 0.15* |
| | | | | | |
| *All* | | | | | |
| | Day 21 | 6.85 ± 0.64 | 6.96 ± 0.56 | 6.93 ± 0.65 | 6.40 ± 0.52 |
| | Difference*²* | 0.13 ± 0.52 | -0.39 ± 0.62 | 0.35 ± 0.56 | -0.61 ± 0.63 |
| | mmol/mol*³* | 1.34 ± 0.15 | 1.38 ± 0.14 | 1.34 ± 0.12 | 1.41 ± 0.14 |
| | | | | | |
| Lathosterol | | | | | |
| *Non-diabetics* | | | | | |
| | Day 21 | 4.65 ± 0.34 | 4.77 ± 0.52 | 4.96 ± 0.50 | 3.86 ± 0.22 |
| | Difference² | 0.47 ± 0.40 | 0.49 ± 0.57 | -1.48 ± 0.53 | -1.30 ± 0.27 |
| | mmol/mol³ | 0.88 ± 0.08 | 0.92 ± 0.13 | 0.94 ± 0.09 | 0.86 ± 0.09 |
| | | | | | |
| *Type 2 diabetics* | | | | | |
| | Day 21 | 4.25 ± 0.58 | 5.24 ± 0.47 | 5.03 ± 0.62 | 4.50 ± 0.28 |
| | Difference² | -1.25 ± 0.42 | 0.31 ± 0.59 | -0.16 ± 0.51 | -1.19 ± 0.72 |
| | mmol/mol³ | 0.85 ± 0.13 | 1.06 ± 0.11 | 1.03 ± 0.12 | 0.94 ± 0.05 |
| | | | | | |
| *All* | | | | | |
| | Day 21 | 4.47 ± 0.32 | 4.98 ± 0.35 | 4.99 ± 0.38 | 4.15 ± 0.18 |
| | Difference*²* | -0.33 ± 0.33*^{ab}* | 0.17 ± 0.40 *^{a}* | -0.86 ± 0.38 *^{ab}* | -1.25 ± 10.36 *^{b}* |
| | mmol/mol*³* | 0.86 ± 0.07 | 0.98 ± 0.09 | 0.98 ± 0.07 | 0.89 ± 0.06 |
| | | | | | |
| β-sitosterol | | | | | |
| *Non-diabetics* | | | | | |
| | Day 21 | 4.20 ± 0.61 | 6.08 ± 1.10 | 3.57 ± 0.51 | 4.68 ± 0.63 |
| | Difference² | 0.07 ± 0.37 | 1.02 ± 0.51 | -0.31 ± 0.23 | 0.82 ± 0.39 |
| | mmol/mol³ | 0.78 ± 0.11 | 1.25 ± 0.28 | 0.69 ± 0.11 | 1.06 ± 0.18 |
| | | | | | |
| *Type 2 diabetics* | | | | | |
| | Day 21 | 4.92 ± 0.76 | 4.84 ± 1.04 | 4.09 ± 0.80 | 4.77 ± 0.85 |
| | Difference² | 0.34 ± 0.42 | 0.30 ± 0.37 | -0.13 ± 0.42 | 0.30 ± 0.35 |
| | mmol/mol³ | 0.99 ± 0.18 | 0.95 ± 0.20 | 0.82 ± 0.16 | 0.99 ± 0.17 |
| | | | | | |
| *All* | | | | | |
| | Day 21 | 4.53 ± 0.47*^{ab}* | 5.50 ± 0.76*^{a}* | 3.81 ± 0.45*^{b}* | 4.72 ± 0.51*^{ab}* |
| | Difference*²* | 0.20 ± 0.28 | 0.68 ± 0.32 | -0.22 ± 0.23 | 0.57 ± 1.32 |
| | mmol/mol*³* | 0.87 ± 0.10^{ab} | 1.11 ± 0.18*^{a}* | 0.75 ± 0.09*^{b}* | 1.03 ± 0.12*^{ab}* |

| | | | | | |
|---|---|---|---|---|---|
| *¹*Values are expressed as mean ± SE. Values carrying different superscript letters indicate a significant difference between groups (p<0.05). Percent change is based on individual data. Percent change relative to control is based on the mean of day 21. *P<0.05; significant difference from non diabetic group. *²*Difference between day 0 and day 21. *³*Plant sterol per 1 mol cholesterol. | | | | | |

**Table 8. Plasma insulin levels at day 0 and day 21 of each treatment period¹**

| Insulin (µlU/mL) | | Control | Plant sterols | Glucomannan | Combination |
|---|---|---|---|---|---|
| *Non-diabetics* | | | | | |
| | Day 0 | 21.44 ± 2.61 | 21.30 ± 2.76 | 19.7 ± 1.39 | 22.31 ± 2.47 |
| | Day 21 | 22.08 ± 1.88 | 20.85 ± 1.52 | 19.21 ± 1.76 | 21.90 ± 2.53 |
| | % Change | 13.42 ± 10.15 | 10.81 ± 10.49 | -3.07 ± 4.96 | 1.91 ± 8.68 |
| | % Relative to C2 | | -0.74 ± 6.72 | -78.28 ± 36.08 | -1.07 ± 6.60 |
| | | | | | |
| *Type 2 diabetics* | | | | | |
| | Day 0 | 39.31 ± 3.48*** | 37.68 ± 4.01** | 39.03 ± 4.49*** | 36.21 ± 4.68** |
| | Day 21 | 39.39 ± 4.00*** | 40.18 ± 4.80*** | 42.59 ± 4.94*** | 34.51 ± 4.32* |
| | % Change | 2.70 ± 6.82 | 15.09 ± 15.80 | 19.54 ± 16.45 | 5.66 ± 14.50 |
| | % Relative to C² | | 4.74 ± 11.25 | -80.03 ± 35.65 | -11.27 ± 6.98 |
| | | | | | |
| *All* | | | | | |
| | Day 0 | 29.45 ± 2.68 | 28.64 ± 2.78 | 28.37 ± 2.78 | 28.54 ± 2.78 |
| | Day 21 | 29.84 ± 2.60 | 29-51 ± 2.90 | 29.69 ± 3.23 | 27.55 ± 2.62 |
| | % Change | 8.62 ± 6.36 | 12.73 ± 8.98 | 7.07 ± 7.99 | 3.59 ± 7.92 |
| | % Relative to C² | | 1.71 ± 6.16*^{b}* | -79.7 ± 25.08*^{a}* | -5.64 ± 4.81*^{b}* |

| | | | | | |
|---|---|---|---|---|---|
| *¹*Values are expressed as mean ± SE. Values carrying different superscript letters indicate a significant difference between treatments (p<0.05). Percent change is based on individual data. Percent change relative to control is based on the mean of day 21. *P<0.05; **P<0.01; ***P<0.001; significant difference from non diabetic group. *²*Control treatment. | | | | | |

**Table 9. Serum fructosamine levels at day 0 and day 21 of each treatment period¹**

| Fructosamine (umol/g) | | Control | Plant sterols | Glucomannan | Combination |
|---|---|---|---|---|---|
| *Non-diabetics* | | | | | |
| | Day 0 | 3.38 ± 0.07 | 3.39 ± 0.07 | 3.44 ± 0.07 | 3.48 ± 0.09 |
| | Day 21 | 3.40 ± 0.08 | 3.38 ± 0.06 | 3.42 ± 0.06 | 3.40 ± 0.08 |
| | % Change | 0.15 ± 0.85 | 0.08 ± 1.42 | -0.33 ± 1.10 | -2.02 ± 1.21 |
| | % Relative to ²C | | 0.65 ± 1.83 | 1.26 ± 1.02 | 1.39 ± 1.58 |
| | | | | | |
| *Type 2 diabetics* | | | | | |
| | Day 0 | 4.08 ± 6.18*** | 4.00 ± 0.19** | 4.05 ± 0.16*** | 4.10 ± 0.17** |
| | Day 21 | 4.01 ± 0.16** | 3.94 ± 0.18** | 3..92 ± 0.14*** | 3.95 ± 0.13** |
| | % Change | -1.42 ± 1.41 | -1.32 ± 1.04 | -2.87 ± 0.92 | -3.28 ± 1.83 |
| | % Relative to ²C | | -1.68 ± 1.79 | -1.86 ± 0.92 | -0.91 ± 2.46 |
| | | | | | |
| *All* | | | | | |
| | Day 0 | 3.69 ± 0.11 3.66 ± 0.11 | | 3.71 ± 0.10 | 3-76 ± 0.11 |
| | Day 21 | 3.68 ± 0.10 | 3.63 ± 0.10 | 3.65 ± 6.08 | 3.65 ± 0.09 |
| | % Change | -0.58 ± 0.80 | -0.55 ± 0.91 | -1.47 ± 0.76 | -2.58 ± 1.04 |
| | % Relative to ²C | | -0.43 ± 1.28 | -0.19 ± 0.74 | 0.32 ± 1.41 |

| | | | | | |
|---|---|---|---|---|---|
| *¹*Values are expressed as mean ± SE. Values carrying different superscript letters indicate a significant difference between treatments (p<0.05). Percent change is based on individual data. Percent change relative to control is based on the mean of day 21. **P<0.01; ***P<0.001; significant difference from non diabetic group. *²*Control treatment | | | | | |

### References:

1. Levi RI Declining Mortality in Coronary Hear Diseases Atherosclerosis 1981 1 312-325
2. Law M.R., Wald N.J., Wu., Hacksaw ZA., Bailey A.; Systemic underestimation of association between serum cholesterol concentration and ischemic heart disease in observational studies: Data from BUPA Study; Br. Med. J. 1994; 308:363-366
3. Law M.R., Wald N.J., Thompson S.G.; By how much and how quickly does reduction in serum cholesterol concentration lower risk of ischemic heart disease? Br. Med. J. 1994; 308:367-373
4. La Rosa J.C., Hunninghake D.. Bush D. et al.; The cholesterol facts: A summary of the evidence relating to dietary fats, serum cholesterol and coronary heart disease: A joint statement by the American Heart Association and the National Heart, Lung and Blood Institute. Circulation 1990; 81:1721-1733
5. Havel R.J., Rapaport E.. Drug Therapy: Management of Primary Hyperlipidemia. New England Journal of Medicine, 1995; 332:1491-1498
6. Lees A.M., Mok H.Y.I., Lees R.S., McCluskey M.A., Grundy S.M. Plant sterols as cholesterol-lowering agents: clinical trials in patients with hypercholesterolemia and studies of sterol balance. Atherosclerosis 1977; 28: 325-338
7. New Eng. J. Med., 336, 973-9 (1999)
8. J. Path. 181, 93-9 (1997)
9. Liese AD et al. Development of the Insulin Resistance Syndrome:An Epidemiological Perspective Epidemiol. Rev 20: 157-172, 1998
10. Trevisan M. Lui J, Bahsas FB et al: Syndrome X and Mortality: a population based study. Am J Epidemiol 148:958-966, 1998
11. Wing M, Gaskill SP, Haffner SM, Stern MP: Effects of Diabetes and Level of Glycemia on All-Cause and Cardiovascular Mortaility. Diabetes Care 21:1167-1172,1998
12. Gu K, Cowie CC, Harris MI: Mortality in Adults with and without Diabetes in a National Cohort of the US Popluation, 1971-1993 Diabetes Care 21:1138-1145, 1998
13. Savage PJ: Cardiovascular Complications of Diabetes Mellitus: what we know and what we need to know about prevention. Am J. Intern Med. 124:123-126, 1996
14. Kiriyama S, Enishi A, Yoshida A, Suhiyama N and Shimahara H. Hypercholesterolemic activity and molecular weight of Konjak Mannan. Nutri Reports Intl. 6:231-236, 1972
15. Staianno A, Simeone D, Giudice ED et al. Effect of Dietary Fibre Glucomannan on Chronic Constipation in Neurologically impaired children. J. Pediatr 136:41-50, 2000
16. Melga P, Giusto M, Ciuchi E et al. Dietary Fibre in the dietetic therapy of diabetes mellitus. Experimental data with purified glucomannans. Riv. Eur, Sci. Med. Farmacol, 14:367-373. 1992
17. Cesa F, Mariani S, Fava A, et al. The use of vegetable fibres in the treatment of pregnancy related diabetes and/or excessive weight gain during pregnancy. Minerva Ginecol. 42:271-274. 1990
18. Arvill A, Bodin L. Effect of short term ingestion of Kojak glucomannan on serum cholesterol in healthy men. Am J. Clin Nutr 69:30-42, 1999
19. Shima K. Tanaka A, Ikegami H, Tabata M, Sawazaki N, Kumahara Y: Effect of dietary fibre, glucomannan, on absorption of sulfonylurea in man. Horm Metab Res 15:1-3, 1983
20. Hopman WP, Houben PG, Speth PA, Larmers CB. Glucomannan prevents postprandial hypoglycemia in patients with previous gastric surgery. Gut. 1988; 29: 930-934.
21. Scalfi L, Coltorti A, D'Arrigo E, Carandente V, Mazzacano C, Di Palo M, Contaldo F. Effect of dietary fibre on postprandial thermogenesis. Int J Obes. 1987; 11: 95-99.
22. Huang CY, Zhang MY, Peng SS, Hong JR, Wang X, Jiang HJ, Zhang FL, Bai YX, Liang JZ, Yu YR et al. Effect of konjac food on blood glucose level in patients with diabetes. Biomed Environ Sci. 1990; 3: 123-132.

## Claims

1. A composition for incorporation into foods, beverages, nutritional or vitamin supplements, and nutraceuticals, comprising one or more phytosterols and/or phytostanols and additionally, glucomannan, wherein the phytosterol and/or phytostanol in the composition comprises 67.3% sitosterol, 10.8% sitostanol, 8.2% campesterol, 1.6% campestanol and 12.1 % of others.

2. The composition of any one of the preceding claims wherein phytosterol and/or phytostanol is sourced from tall oil pitch.

3. The composition of claim 1 wherein the phytosterols and/or phytostanols are in forms selected from the group consisting of: aliphatic acid esters, aromatic acid esters, phenolic acid esters, cinnamate esters, ferulate esters, phytosterol/phytostanol glycosides, and phytosterol/phytostanol acylglycosides.

4. Use of a combination comprising one or more phytosterols and/or phytostanols and additionally glucomannan in the manufacture of a prophylactic or medicament for treating or preventing atherosclerosis, hyperlipidemic conditions, dyslipidemia, or type II diabetes, hypoalphalipoproteinemia, hypertension, hypercholesterolemia and visceral obesity in an animal, wherein the phytosterol and/or phytostanol in the composition comprises 67.3% sitosterol, 10.8% sitostanol, 8.2% campesterol, 1.6% campestanol and 12.1% of others.

5. The use of claim 4 wherein the amount of phytosterol and/or phytostanol is at least 0.8 g/day and the amount of glucomannan is up to 13 g/day based on a 70 kg individual human.

6. The use of claim 5 wherein the amount of phytosterol and/or phytostanol is 1-3 g/day and the amount of glucomannan used is 2-10 g/day.

7. The use of any one of claim 5 4 to 6 for treating or preventing cardiovascular disease and/or its underlying conditions in an animal.

8. The use of any one of claims 4 to 7 wherein phytosterol and/or phytostanol and glucomannan used is in the form of a composition of any one of claims 1-3.

9. The use of any of claims 4 to 8 wherein the animal is human.

10. The use according to claim 9 in which the human has type II diabetes.

11. The use according to any one of claims 4 to 10 in which the treatment further achieves at least one of the following therapeutic goals in the animal: lowering serum LDL cholesterol, increasing serum HDL cholesterol and decreasing serum triglycerides.

12. The use according to any one of claims 4 to 11 in which the treatment decreases weight gain or increases weight loss in the animal.

13. A product which is a functional food, a beverage, a nutritional or vitamin supplement or a nutraceutical comprising the composition according to any one of claims 1 to 3.

14. A product according to claim 13 which is a food.

15. A product according to claim 13 which is a beverage.

## Patentansprüche

1. Zusammensetzung zur Integration in Nahrungsmittel, Getränke, Nahrungsergänzungsmittel oder Vitaminpräparate sowie Nutraceuticals, die ein oder mehrere Phytosterol(e) und/oder Phytostanol(e) und zusätzlich Glucomannan umfasst, wobei das Phytosterol und/oder Phytostanol in der Zusammensetzung 67,3 % Sitosterol, 10,8 % Sitostanol, 8,2 % Campesterol, 1,6 % Campestanol und 12,1 % andere umfasst.

2. Zusammensetzung nach einem der vorangehenden Ansprüche, wobei das Phytosterol und/oder Phytostanol aus Tallölpech gewonnen wird.

3. Zusammensetzung nach Anspruch 1, wobei die Phytosterole und/oder Phytostanole in Formen vorliegen, die ausgewählt sind aus der Gruppe bestehend aus Estern aliphatischer Säuren, Estern aromatischer Säuren, Phenolsäureestern, Cinnamatestern, Ferulatestern, Phytosterol-/Phytostanolglykosiden und Phytosterol-/Phytostanolacylglykosiden.

4. Verwendung einer Kombination, die ein oder mehrere Phytosterol(e) und/oder Phytostanol(e) und zusätzlich Glucomannan umfasst, bei der Herstellung eines Prophylaktikums oder Medikaments zur Behandlung oder Vorbeugung von Arteriosklerose, hyperlipidämischen Zuständen, Dyslipidämie oder Diabetes Typ II, Hypoalphalipoproteinämie, Hypertonie, Hypercholesterinämie und Stammfettsucht bei einem Tier, wobei das Phytosterol und/oder Phytostanol in der Zusammensetzung 67,3 % Sitosterol, 10,8 % Sitostanol, 8,2 % Campesterol, 1,6 % Campestanol und 12,1 % andere umfasst.

5. Verwendung nach Anspruch 4, wobei die Menge an Phytosterol und/oder Phytostanol mindestens 0,8 g/Tag beträgt und die Menge an Glucomannan bis zu 13 g/Tag beträgt, bezogen auf einen 70 kg schweren Menschen.

6. Verwendung nach Anspruch 5, wobei die Menge an Phytosterol und/oder Phytostanol 1-3 g/Tag beträgt und die Menge an verwendetem Glucomannan 2-10 g/Tag beträgt.

7. Verwendung nach einem der Ansprüche 4 bis 6 zur Behandlung oder Vorbeugung von Herz-Kreislauf-Erkrankungen und/oder der diesen zugrunde liegenden Zustände bei einem Tier.

8. Verwendung nach einem der Ansprüche 4 bis 7, wobei das verwendetePhytosterol und/oder Phytostanol sowie das verwendete Glucomannan in der Form einer Zusammensetzung nach einem der Ansprüche 1-3 vorliegen.

9. Verwendung nach einem der Ansprüche 4 bis 8, wobei das Tier ein Mensch ist.

10. Verwendung nach Anspruch 9, wobei der Mensch an Diabetes Typ II leidet.

11. Verwendung nach einem der Ansprüche 4 bis 10, wobei durch die Behandlung ferner mindestens eines der folgenden therapeutischen Ziele bei dem Tier erreicht wird: Senkung des LDL-Cholesterins im Serum, Erhöhung des HDL-Cholesterins im Serum und Verminderung der Serumtriglyceride.

12. Verwendung nach einem der Ansprüche 4 bis 11, wobei die Behandlung bei dem Tier die Gewichtszunahme vermindert oder die Gewichtsabnahme erhöht.

13. Produkt, bei dem es sich um ein funktionelles Lebensmittel, ein Getränk, ein Nahrungsergänzungsmittel oder Vitaminpräparat oder ein Nutraceutical handelt, das die Zusammensetzung nach einem der Ansprüche 1 bis 3 umfasst.

14. Produkt nach Anspruch 13, bei dem es sich um ein Nahrungsmittel handelt.

15. Produkt nach Anspruch 13, bei dem es sich um ein Getränk handelt.

## Revendications

1. Composition destinée à être incorporée dans des produits alimentaires, des boissons, des compléments nutritionnels ou vitaminiques, et des produits neutraceutiques comportant un ou plusieurs phytostérols et/ou phytostanols et, additionnellement, un glucomannan, dans laquelle le phytostérol et/ou phytostanol dans la composition comporte 67,3 % de sitostérol, 10,8 % de sitostanol, 8,2 % de campestérol, 1,6 % de campestanol et 12,1 % d'autres produits.

2. Composition selon l'une quelconque des revendications précédentes dans laquelle le phytostérol et/ou phytostanol est extrait de brai de tall-oil.

3. Composition selon la revendication 1 dans laquelle les phytostérols et/ou phytostanols sont sous des formes choisies dans le groupe constitué par : des esters d'acide aliphatique, des esters d'acide aromatique, des esters d'acide phénolique, des esters cinnamate, des esters de ferulate, des glycosides de phytostérol/phytostanol, et des glycosides acylés de phytostérol/phytostanol.

4. Utilisation d'une préparation combinée comportant un ou plusieurs phytostérols et/ou phytostanols et, additionnellement, un glucomannan dans la fabrication d'un produit prophylactique ou d'un médicament pour traiter ou prévenir l'athérosclérose, des états hyperlipidémiques, la dyslipidémie, ou le diabète de type II, l'hypoalphalipoprotéinémie, l'hypertension, l'hypercholestérolémie et l'obésité viscérale chez un animal, dans laquelle le phytostérol et/ou phytostanol dans la composition comporte 67,3 % de sitostérol, 10,8 % de sitostanol, 8,2 % de campestérol, 1,6 % de campestanol et 12,1 % d'autres produits.

5. Utilisation selon la revendication 4 dans laquelle la quantité de phytostérol et/ou phytostanol est d'au moins 0,8 g/jour et la quantité de glucomannan s'élève jusqu'à 13 g/jour en se basant sur un être humain de 70 kg.

6. Utilisation selon la revendication 5 dans laquelle la quantité de phytostérol et/ou phytostanol est de 1 à 3 g/jour et la quantité de glucomannan utilisée est de 2 à 10 g/jour.

7. Utilisation selon l'une quelconque des revendications 4 à 6 pour traiter ou prévenir les maladies cardio-vasculaires et/ou leurs affections sous-jacentes chez un animal.

8. Utilisation selon l'une quelconque des revendications 4 à 7 dans laquelle le phytostérol et/ou phytostanol et glucomannan utilisés sont sous la forme d'une composition selon l'une quelconque des revendications 1 à 3.

9. Utilisation selon l'une quelconque des revendications 4 à 8 dans laquelle l'animal est un être humain.

10. Utilisation selon la revendication 9 dans laquelle l'être humain souffre d'un diabète de type II.

11. Utilisation selon l'une quelconque des revendications 4 à 10 dans laquelle le traitement atteint en outre au moins un des buts thérapeutiques suivants chez l'animal : abaisser le cholestérol LDL sérique, augmenter le cholestérol HDL sérique et réduire les triglycérides sériques.

12. Utilisation selon l'une quelconque des revendications 4 à 11 dans laquelle le traitement réduit le gain de poids ou augmente la perte de poids chez l'animal.

13. Produit qui est un produit alimentaire fonctionnel, une boisson, un complément nutritionnel ou vitaminique ou un produit neutraceutique, comportant la composition selon l'une quelconque des revendications 1 à 3.

14. Produit selon la revendication 13 qui est un produit alimentaire.

15. Produit selon la revendication 13 qui est une boisson.
